# EUROPEAN PATENT APPLICATION

(11) **EP 3 243 468 A1**
(43) Date of publication of application: **15.11.2017**
(21) Application number: 16827452.0
(22) Date of filing: 03.02.2016
(51) Int. Cl.: A61B 18/00

(54) **ULTRASONIC PROBE FOR ARTHROSCOPIC SURGERY AND ULTRASONIC PROBE UNIT**

(30) Priority: 23.07.2015 US 201562196158 P
(71) Applicant: Olympus Corporation, Hachioji-shi, Tokyo 192-8507 (JP)
(72) Inventor: YOSHIMINE, Hideto, Hachioji-shi, Tokyo 192-8507 (JP)
(74) Representative: Gunzelmann, Rainer
(86) International application number: PCT/JP2016/053246
(87) International publication number: WO 2017/013886

(57) **Abstract**

An ultrasonic probe for arthroscopic surgery includes: a narrowing portion which is continuous with a distal side of a probe main body and whose sectional area vertical to a longitudinal axis decreases from a proximal side toward a distal side; a bend extending portion provided on the distal side of the narrowing portion and extending in a state of bending in an intersecting direction to the longitudinal axis when the intersecting direction intersecting the longitudinal axis is prescribed; and a treatment portion provided on the distal side of the bend extending portion, and including a cutting region configured to cut a bone or a cartilage by use of the ultrasonic vibration in the joint at a position more away from the longitudinal axis than the bend extending portion in the intersecting direction.

## Description

### Technical Field

The present invention relates to an ultrasonic probe and an ultrasonic probe unit for arthroscopic surgery which are used for an operation in a joint and transmit ultrasonic vibration.

### Background Art

In Patent Literature 1, there is disclosed an ultrasonic treatment instrument including an ultrasonic probe (an ultrasonic horn). In this ultrasonic treatment instrument, ultrasonic vibration generated in a vibration generating section (an ultrasonic vibration mechanism) is transmitted from a proximal side to a distal side in the ultrasonic probe. In a distal portion of the ultrasonic probe, a scalpel portion is formed as a cutting region. In a state where the cutting region is in contact with a treatment target, the ultrasonic vibration is transmitted to the scalpel portion, whereby the treatment target (e.g., a bone or the like) is cut.

### Citation List

### Patent Literature

Patent Literature 1: Jpn. Pat. Appln. KOKAI Publication No. 2003-116870

### Summary of Invention

### Technical Problem

In a joint such as a knee joint, a shoulder joint or an elbow joint, it is necessary to cut a treatment target such as a bone or a cartilage in a very narrow space. According to a constitution of Patent Literature 1 mentioned above, in a narrow space of the joint or the like, for example, a region other than a scalpel portion in an ultrasonic probe interferes with a tissue or the like other than the treatment target, whereby there is the possibility that the scalpel portion of a cutting region does not appropriately come in contact with the treatment target.

The present invention has been developed to solve the above problems, and an object thereof is to provide an ultrasonic probe and an ultrasonic probe unit for arthroscopic surgery in which a cutting region suitably comes in contact with a treatment target even in a narrow space of a joint or the like.

### Solution to Problem

According to an aspect of the present invention to solve the object, there is provide an ultrasonic probe for arthroscopic surgery which is for use in an operation of a joint and which is configured to transmit ultrasonic vibration from a proximal side to a distal side thereof, includes: a probe main body which extends from the proximal side to the distal side along a linear longitudinal axis and to which an ultrasonic transducer to generate the ultrasonic vibration is connected to the proximal side; a narrowing portion which is continuous with the distal side of the probe main body and whose sectional area vertical to the longitudinal axis decreases from the proximal side toward the distal side; a bend extending portion which is provided on the distal side of the narrowing portion and which extends in a state of bending in an intersecting direction to the longitudinal axis when the intersecting direction intersecting the longitudinal axis is prescribed; and a treatment portion which is provided on the distal side of the bend extending portion, and which includes a cutting region that is configured to cut a bone or a cartilage by use of the ultrasonic vibration in the joint at a position more away from the longitudinal axis than the bend extending portion in the intersecting direction, wherein in projection seen from the distal side, the narrowing portion, the bend extending portion and the treatment portion are arranged in a range inner than a minimum inner diameter of a sheath into which the ultrasonic probe is inserted.

### Advantageous Effect of the Invention

According to the present invention, it is possible to provide an ultrasonic probe for arthroscopic surgery in which a cutting region suitably comes in contact with a treatment target even in a narrow space of a joint or the like.

### Brief Description of Drawings

FIG. 1 is a schematic view showing an ultrasonic treatment system according to a first embodiment;
FIG. 2 is a schematic view showing a constitution of a vibrating body unit according to the first embodiment;
FIG. 3 is a schematic view of a distal portion of an ultrasonic probe according to the first embodiment which is seen from one side in a width direction;
FIG. 4 is a schematic view of the distal portion of the ultrasonic probe according to the first embodiment which is seen from the side of a second intersecting direction;
FIG. 5 is a cross-sectional view taken along the V-V line of FIG. 3;
FIG. 6 is a schematic view of a cutting region of a treatment portion according to the first embodiment which is seen from the second intersecting direction side;
FIG. 7 is a schematic view of the treatment portion according to the first embodiment seen from a direction of an arrow VII of FIG. 6;
FIG. 8 is a schematic view of a sheath and the ultrasonic probe according to the first embodiment seen from a distal side thereof;
FIG. 9A is a schematic view showing one example of a state where the treatment portion of the ultrasonic probe according to the first embodiment is made to access a space of a joint cavity to cut a treatment target with the cutting region of the treatment portion;
FIG. 9B is a schematic view showing one example of a state where a treatment portion of an ultrasonic probe according to a reference example is made to access a space of a joint cavity to cut a treatment target with a cutting region of the treatment portion;
FIG. 10A is a schematic view showing one example of a state where the treatment portion of the ultrasonic probe according to the first embodiment is made to access a position inner than a position shown in FIG. 9A in the space of the joint cavity to cut the treatment target with the cutting region of the treatment portion;
FIG. 10B is a schematic view showing one example of a state where the treatment portion of the ultrasonic probe according to the reference example is made to access a position inner than a position shown in FIG. 9B in a space of a joint cavity to cut a treatment target with a cutting region of the treatment portion;
FIG. 11 is a schematic view showing a treatment target cut with the cutting region according to the first embodiment;
FIG. 12 is a schematic view of a sheath and an ultrasonic probe according to a modification of the first embodiment which are seen from a distal side thereof;
FIG. 13 is a schematic view of a distal portion of an ultrasonic probe according to a second embodiment which is seen from one side in a width direction;
FIG. 14 is a schematic view of the distal portion of the ultrasonic probe according to the second embodiment which is seen from the side of a second intersecting direction;
FIG. 15 is a cross-sectional view taken along the XV-XV line of FIG. 13;
FIG. 16 is a schematic view of a treatment portion according to the second embodiment which is seen from a direction of an arrow VII of FIG. 6;
FIG. 17 is a schematic view of a sheath and an ultrasonic probe according to a modification of the second embodiment which are seen from a distal side thereof;
FIG. 18 is a schematic view of a distal portion of an ultrasonic probe according to a third embodiment which is seen from one side in a width direction;
FIG. 19 is a schematic view of the distal portion of the ultrasonic probe according to the third embodiment which is seen from the side of a second intersecting direction;
FIG. 20 is a schematic view of a cutting region of a treatment portion according to the third embodiment which is seen from one side in a width direction;
FIG. 21 is a schematic view of a sheath and the ultrasonic probe according to the third embodiment which are seen from a distal side thereof;
FIG. 22 is a schematic view of a sheath and an ultrasonic probe according to a modification of the third embodiment which are seen from a distal side thereof;
FIG. 23 is a schematic view of a distal portion of an ultrasonic probe according to a fourth embodiment which is seen from one side in a width direction;
FIG. 24 is a schematic view of the distal portion of the ultrasonic probe according to the fourth embodiment which is seen from the side of a second intersecting direction;
FIG. 25 is a schematic view of a cutting region of a treatment portion according to the fourth embodiment which is seen from the one side in the width direction;
FIG. 26 is a schematic view of a sheath and the ultrasonic probe according to the fourth embodiment which are seen from a distal side thereof;
FIG. 27 is a schematic view of a distal portion of an ultrasonic probe according to a fifth embodiment which is seen from one side in a width direction;
FIG. 28 is a schematic view of the distal portion of the ultrasonic probe according to the fifth embodiment which is seen from the side of a second intersecting direction;
FIG. 29 is a cross-sectional view taken along the XXIX-XXIX line of FIG. 27; and
FIG. 30 is a schematic view of the distal portion of the ultrasonic probe according to the fifth embodiment which is seen from the one side in the width direction.

### Brief Description of Embodiments

### (First Embodiment)

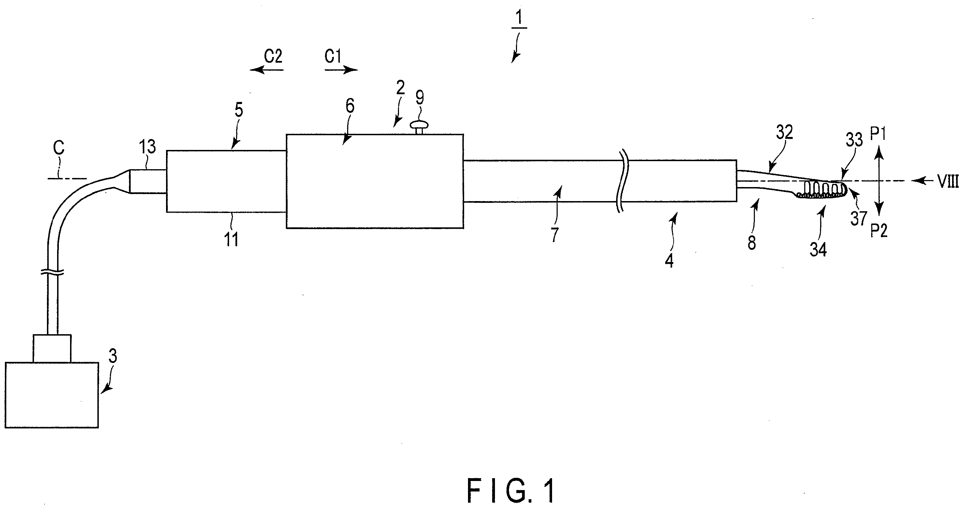

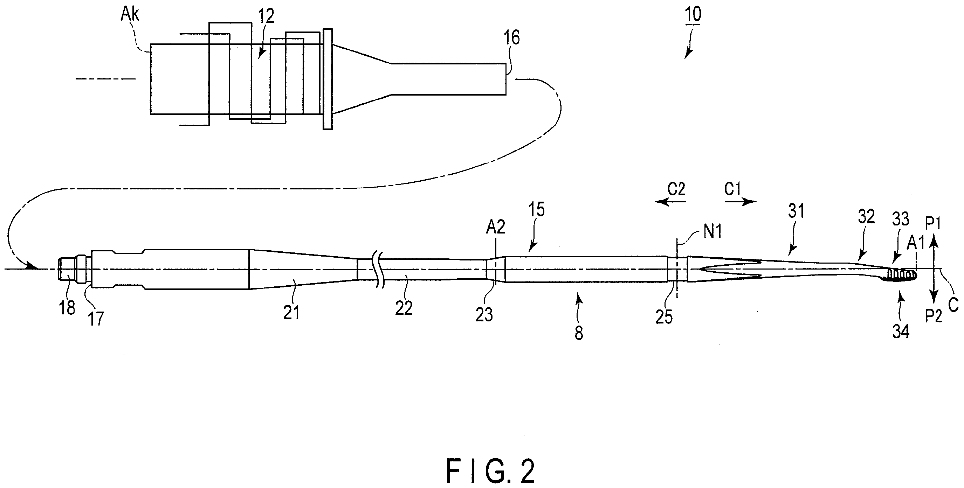

A first embodiment will be described with reference to FIG. 1 to FIG. 12. FIG. 1 is a view showing an ultrasonic treatment system 1 of the present embodiment. FIG. 2 is a view showing a constitution of a vibrating body unit 10 including an ultrasonic probe 8 and an ultrasonic transducer 12 which will be described later. As shown in FIG. 1, the ultrasonic treatment system 1 includes an ultrasonic treatment instrument (a hand piece) 2, an energy controller 3, and a transducer unit 5. The ultrasonic treatment instrument 2 has a substantially linear virtual longitudinal axis C. Here, one side of a direction along the longitudinal axis C (a longitudinal direction) is a distal side (an arrow C1 side), and a side opposite to the distal side is a proximal side (an arrow C2 side). Furthermore, the ultrasonic treatment instrument 2 is for use in an operation of arthroscopically cutting a bone or a cartilage in a joint such as a knee joint, a shoulder joint or an elbow joint.

The ultrasonic treatment instrument 2 includes a housing 6 which is able to be held, a sheath 7, and the ultrasonic probe 8 which is used for arthroscopic surgery. The sheath 7 and the ultrasonic probe 8 form an ultrasonic probe unit 4 for the arthroscopic surgery. The housing 6 extends along the longitudinal axis C and the sheath 7 is coupled with the housing 6 from the distal side. The sheath 7 is a hollow member extending along the longitudinal axis C and having the longitudinal axis C as a substantially central axis. The ultrasonic probe (a vibration transmitting member) 8 is inserted through the sheath 7. A distal portion of the ultrasonic probe 8 projects from a distal end of the sheath 7 toward the distal side. Furthermore, an operation button 9 that is an energy operation input section to be operated by an operator is attached to the housing 6.

The transducer unit 5 includes a transducer case 11, and the ultrasonic transducer 12 (see FIG. 2) provided in the transducer case 11. The transducer case 11 is coupled with the housing 6 from the proximal side. Furthermore, in the housing 6, the ultrasonic transducer 12 is connected to the ultrasonic probe 8 from the proximal side. The transducer unit 5 is connected to the energy controller 3 via a cable 13. The energy controller 3 includes an electric power source, a conversion circuit that converts an electric power from the electric power source into electric energy to be supplied to the ultrasonic transducer 12, a processor or the like (a control section) including a CPU (central processing unit), an ASIC (application specific integrated circuit) or the like, and a storage medium such as a memory. The energy controller 3 detects input of the operation in the operation button 9 to output the electric energy to the ultrasonic transducer 12.

When the electric energy is supplied to the ultrasonic transducer 12, ultrasonic vibration is generated in the ultrasonic transducer 12. Then, the generated ultrasonic vibration is transmitted to the ultrasonic probe 8, and in the ultrasonic probe 8, the ultrasonic vibration is transmitted from the proximal side to the distal side. In this case, the vibrating body unit 10 constituted of the ultrasonic transducer 12 and the ultrasonic probe 8 vibrates (longitudinally vibrates) at any frequency in a prescribed frequency range. For example, the vibrating body unit 10 is designed in a state of transmitting the ultrasonic vibration therethrough, thereby performing the longitudinal vibration at 47 kHz, and the vibrating body unit actually longitudinally vibrates at any frequency in the frequency range of 46 kHz or more and 48 kHz or less. Furthermore, in the state where the vibrating body unit 10 longitudinally vibrates at any frequency in the prescribed frequency range as shown in FIG. 2, a vibration antinode A1 of the longitudinal vibration is positioned in the distal portion of the ultrasonic probe 8 and a vibration antinode Ak of the longitudinal vibration is positioned at a proximal end of the ultrasonic transducer 12. Here, the vibration antinode A1 is positioned most distally among vibration antinodes Ai (i = 1, 2, ..., k) of the longitudinal vibration, and the vibration antinode Ak is positioned most proximally among the vibration antinodes Ai.

The ultrasonic transducer 12 extends along the substantially linear virtual longitudinal axis C that is the substantially central axis. At a distal end of the ultrasonic transducer 12, a transducer contact surface 16 is formed. The ultrasonic probe 8 includes a probe main body 15 extending along the substantially linear longitudinal axis C. The probe main body 15 extends along the longitudinal axis C that is the substantially central axis. At a proximal end of the probe main body 15, a probe contact surface 17 is formed. Furthermore, in the ultrasonic probe 8, there is provided an engaging protrusion 18 projecting from the probe contact surface 17 (the proximal end of the probe main body 15) to the proximal side. The engaging protrusion 18 engages with an engagement groove (not shown) provided in the ultrasonic transducer 12 (e.g., an external thread of the engaging protrusion 18 is screwed into an internal thread of the engagement groove), whereby the ultrasonic probe 8 is connected to the distal side of the ultrasonic transducer 12. In other words, the ultrasonic transducer 12 that generates the ultrasonic vibration is connected to the proximal side of the probe main body 15. In a state where the ultrasonic probe 8 is connected to the ultrasonic transducer 12, the probe contact surface 17 of the probe main body 15 contacts the transducer contact surface 16 of the ultrasonic transducer 12, and the ultrasonic vibration is transmitted to the ultrasonic probe 8 (the probe main body 15) from the ultrasonic transducer 12 through the transducer contact surface 16 and the probe contact surface 17.

The probe main body 15 includes a horn 21, a sectional area uniform portion 22 provided on the distal side of the horn 21 and having a uniform sectional area, a sectional area increasing portion 23 provided on the distal side of the sectional area uniform portion 22, and a supported portion 25 provided on the distal side of the sectional area increasing portion 23. A sectional area of the horn 21 which is vertical to the longitudinal axis C decreases from the proximal side toward the distal side. In a longitudinally vibrating state of the vibrating body unit 10 at any frequency in the prescribed frequency range (e.g., a range of 46 kHz or more and 48 kHz or less), each vibration antinode Ai of the longitudinal vibration is positioned away from the horn 21. Consequently, in the horn 21, an amplitude of the longitudinal vibration is enlarged. A sectional area of the sectional area increasing portion 23 which is vertical to the longitudinal axis C increases from the proximal side toward the distal side. In the longitudinally vibrating state of the vibrating body unit 10 at any frequency in the prescribed frequency range, a vibration antinode A2 of the longitudinal vibration is positioned in the sectional area increasing portion 23. Consequently, in the sectional area increasing portion 23, the amplitude of the longitudinal vibration hardly decreases. In the longitudinally vibrating state of the vibrating body unit 10 at any frequency in the prescribed frequency range, for example, when the longitudinal vibration having an amplitude of 18 µm is transmitted to a proximal end of the probe main body 15 (the probe contact surface 17), the amplitude of the longitudinal vibration is 80 µm at the vibration antinode A1 positioned in the sectional area increasing portion 23. It is to be noted that the vibration antinode A2 is positioned secondly distally among the vibration antinodes Ai of the longitudinal vibration.

The supported portion 25 is formed into a groove shape dented on an inner peripheral side along the whole periphery around the longitudinal axis C. An elastic member (not shown) having electric insulating properties and heat resisting properties is attached to an outer peripheral surface of the supported portion 25. In the supported portion 25, the ultrasonic probe 8 is supported by the sheath 7 via the elastic member. In the longitudinally vibrating state of the vibrating body unit 10 at any frequency in the prescribed frequency range (the range of 46 kHz or more and 48 kHz or less), a vibration node N1 of the longitudinal vibration is positioned in the supported portion 25. Here, the vibration node N1 is positioned most distally among vibration nodes Nj (j = 1, 2, ..., k-1) of the longitudinal vibration. The distal end of the sheath 7 is positioned on the distal side of the supported portion 25. Consequently, in the longitudinally vibrating state of the vibrating body unit 10 at any frequency in the prescribed frequency range, the most distal vibration node N1 is positioned in the sheath 7.

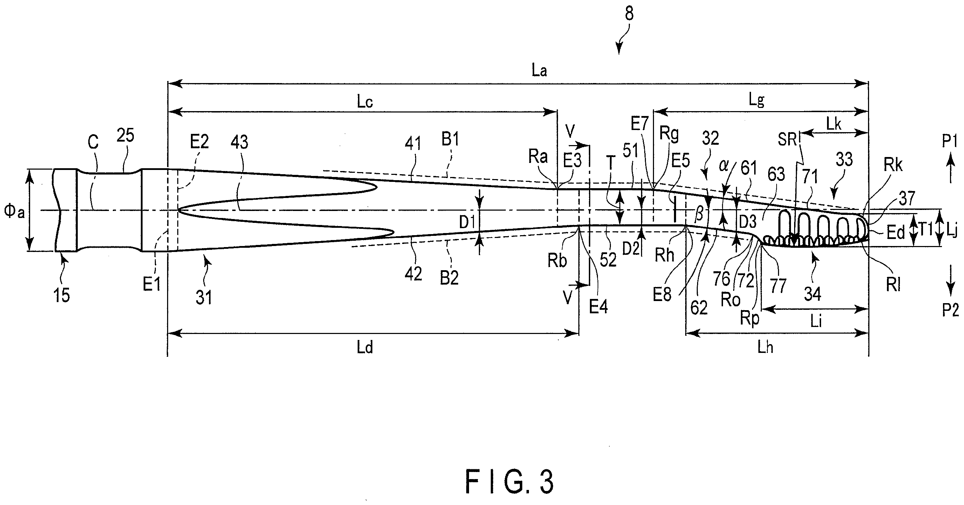

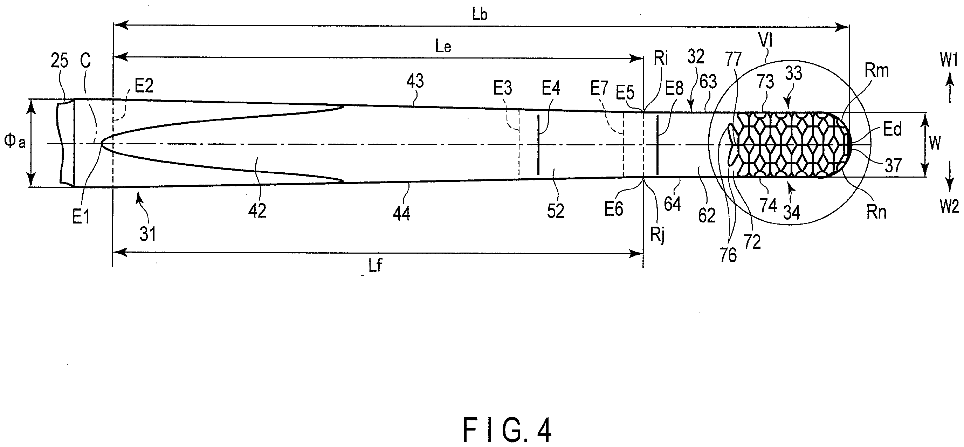

FIG. 3 and FIG. 4 are views showing a constitution of the distal portion of the ultrasonic probe 8. Here are prescribed a first intersecting direction (a direction of an arrow P1) that is a certain direction intersecting (being substantially vertical to) the longitudinal axis C, and a second intersecting direction (a direction of an arrow P2) opposite to the first intersecting direction (a first vertical direction). Furthermore, there are prescribed width directions (directions of an arrow W1 and an arrow W2) of the ultrasonic probe 8 which are substantially vertical to (intersect) the first intersecting direction (the first vertical direction) and the second intersecting direction (a second vertical direction). Each of FIG. 2 and FIG. 3 is a view of the ultrasonic probe 8 seen from one side (e.g., an arrow W1 side shown in FIG. 4) in the width direction, and FIG. 4 is a view of the ultrasonic probe 8 seen from a second intersecting direction P2 side.

As shown in FIG. 2 to FIG. 4, the ultrasonic probe 8 includes a narrowing portion 31 continuous with the distal side of the probe main body 15, and a bend extending portion 32 provided on the distal side of the narrowing portion 31. The bend extending portion 32 extends in a state of bending to the second intersecting direction P2 side of the longitudinal axis C. A treatment portion 33 that treats a treatment target is provided on the distal side of the bend extending portion 32. The treatment portion 33 includes a distal outer surface 37 in the form of a curved surface that forms a distal end Ed of the ultrasonic probe 8. The treatment portion 33 has a cutting region 34 that cuts a bone or a cartilage by use of the ultrasonic vibration in a joint. The cutting region 34 is provided at a position more away from the longitudinal axis C than the bend extending portion 32 in the second intersecting direction P2. It is to be noted that in this embodiment, the cutting region 34 of the treatment portion 33 is formed into a file shape.

As show in FIG. 3 and FIG. 4, the narrowing portion 31 includes a first narrowing outer surface 41 directed to a first intersecting direction side (an arrow P1 side), a second narrowing outer surface 42 directed to the second intersecting direction side (the arrow P2 side), a third narrowing outer surface 43 directed to one side (the arrow W1 side) in the width direction, and a fourth narrowing outer surface 44 directed to the other side (an arrow W2 side) in the width direction. Each of the narrowing outer surfaces 41 to 44 approaches the longitudinal axis C from the proximal side toward the distal side along the longitudinal axis C. It is to be noted that a structure is also preferable where in the narrowing outer surfaces 41 to 44, at least one or two narrowing outer surfaces only approach the longitudinal axis C from the proximal side toward the distal side along the longitudinal axis C.

Here, when a cross section vertical to the longitudinal axis C is taken, portions of the first and second narrowing outer surfaces 41 and 42 in the cross section excluding portions around after-mentioned boundary positions E3 and E4 have a substantially equal distance to the longitudinal axis C.

The narrowing portion 31 includes a first relay surface 51 directed to the first intersecting direction side (the arrow P1 side) on the distal side of the first narrowing outer surface 41, and a second relay surface 52 directed to the second intersecting direction side (the arrow P2 side) on the distal side of the second narrowing outer surface 42. It is preferable that the first and second relay surfaces 51 and 52 are parallel or substantially parallel to each other. Furthermore, it is preferable that the first and second relay surfaces 51 and 52 are parallel to the longitudinal axis C. In particular, the first and second relay surfaces 51 and 52 are parallel to the longitudinal axis C between the boundary position E4 and a boundary position E7.

A boundary position E1 (i.e., the distal end of the probe main body 15 and a proximal end of the narrowing portion 31) between the probe main body 15 and the first and second narrowing outer surfaces 41 and 42 of the narrowing portion 31 is positioned on the distal side from the supported portion 25 of the probe main body 15. In a certain example, at the boundary position E1 (i.e., the distal end of the probe main body 15), a sectional shape of the ultrasonic probe 8 vertical to the longitudinal axis C is a round shape having an outer diameter φa of 2.9 mm to 3.8 mm. A boundary position E2 (i.e., the distal end of the probe main body 15 and the proximal end of the narrowing portion 31) between the probe main body 15 and the third and fourth narrowing outer surfaces 43 and 44 of the narrowing portion 31 is positioned on the distal side from the supported portion 25 of the probe main body 15. Here, the boundary position E1 is disposed closer to the proximal side along the longitudinal axis C than the boundary position E2, but may be closer to the distal side, and the position may be at a position equal to a distance (the same dimension) from the distal end Ed of the treatment portion 33 of the ultrasonic probe 8 along the longitudinal axis C to the boundary position E2.

The distal end of the sheath 7 shown in FIG. 1 is positioned on the distal side from the boundary positions E1 and E2 between the probe main body 15 and the narrowing portion 31. Consequently, an outer peripheral side of a proximal portion of the narrowing portion 31 is covered with the sheath 7. However, an outer periphery of a region other than the proximal portion in the narrowing portion 31 and an outer periphery of the bend extending portion 32 are not covered with the sheath 7. Consequently, in the ultrasonic probe 8, the region other than the proximal portion in the narrowing portion 31 and the bend extending portion 32 project from the distal end of the sheath 7 to the distal side.

In a certain example, a dimension La from the distal end Ed of the treatment portion 33 of the ultrasonic probe 8 to the boundary position E1 between the probe main body 15 and the narrowing portion 31 in a direction along the longitudinal axis C (a longitudinal direction) is from 30.3 mm to 32.5 mm. Furthermore, in the certain example, a dimension Lb from the distal end Ed of the treatment portion 33 of the ultrasonic probe 8 to the boundary position E2 between the probe main body 15 and the narrowing portion 31 in the direction along the longitudinal axis C (the longitudinal direction) is from 20 mm to 32 mm.

The boundary position E3 in FIG. 3 and FIG. 4 is determined by a distal end of the first narrowing outer surface 41 and a proximal end of the first relay surface 51. The first narrowing outer surface 41 extends toward the distal side in the direction along the longitudinal axis C from the boundary position (a narrowing start position) E1 between the probe main body 15 and the narrowing portion 31 to the boundary position (a narrowing end position) E3. Consequently, in the first narrowing outer surface 41 between the boundary position E1 and the boundary position E3, a dimension of the narrowing portion 31 from the longitudinal axis C in the first intersecting direction P1 (i. e. , a thickness direction of the narrowing portion 31) decreases from the proximal side toward the distal side. In the certain example, a dimension Lc from the boundary position E1 to the boundary position E3 in the direction along the longitudinal axis C is 18 mm.

The boundary position E4 in FIG. 3 and FIG. 4 is determined by a distal end of the second narrowing outer surface 42 and a proximal end of the first relay surface 52. The second narrowing outer surface 42 extends toward the distal side in the direction along the longitudinal axis C from the boundary position (the narrowing start position) E1 between the probe main body 15 and the narrowing portion 31 to the boundary position (the narrowing end position) E4. Consequently, in the second narrowing outer surface 42 between the boundary position E1 and the boundary position E4, a dimension of the narrowing portion 31 from the longitudinal axis C in the second intersecting direction P2 (i.e., the thickness direction of the narrowing portion 31) decreases from the proximal side toward the distal side. In the certain example, a dimension Ld from the boundary position E1 to the boundary position E4 in the direction along the longitudinal axis C is from 17 mm to 19 mm.

Here, the boundary positions E3 and E4 are different positions along the longitudinal axis C. In other words, the boundary positions E3 and E4 are not positions having the same dimension from the distal end Ed of the treatment portion 33 of the ultrasonic probe 8 along the longitudinal axis C. In particular, the boundary position E3 is disposed closer to the proximal side than the boundary position E4. A curved surface having a suitable radius Ra is formed at the boundary position E3 between the distal end of the first narrowing outer surface 41 and the proximal end of the first relay surface 51. It is to be noted that a curved surface having a suitable radius Rb is formed at the boundary position E4 between the distal end of the second narrowing outer surface 42 and the proximal end of the second relay surface 52.

The third narrowing outer surface 43 extends toward the distal side in the direction along the longitudinal axis C from the boundary position (the narrowing start position) E2 between the probe main body 15 and the narrowing portion 31 to a boundary position (a narrowing end position) E5. Consequently, in the third narrowing outer surface 43 between the boundary position E2 and the boundary position E5, a dimension of the narrowing portion 31 from the longitudinal axis C in a first width direction W1 (i.e., the width direction of the narrowing portion 31) decreases from the proximal side toward the distal side. In the certain example, a dimension Le from the boundary position E2 to the boundary position E5 along the longitudinal axis C is from 11 mm to 23 mm.

The fourth narrowing outer surface 44 extends toward the distal side in the direction along the longitudinal axis C from the boundary position (the narrowing start position) E2 between the probe main body 15 and the narrowing portion 31 to a boundary position (a narrowing end position) E6. Consequently, in the fourth narrowing outer surface 44 between the boundary position E2 and the boundary position E6, a dimension of the narrowing portion 31 from the longitudinal axis C in a second width direction W2 (i.e., the width direction of the narrowing portion 31) decreases from the proximal side toward the distal side. Therefore, a sectional area of the narrowing portion 31 which is vertical to the longitudinal axis C decreases from the proximal side toward the distal side. In the certain example, a dimension Lf from the boundary position E2 to the boundary position E6 along the longitudinal axis C is from 11 mm to 23 mm.

Consequently, the boundary positions E5 and E6 are positions having an equal distance (the same dimension) from the distal end Ed of the treatment portion 33 of the ultrasonic probe 8 along the longitudinal axis C. Therefore, a center of gravity of the narrowing portion 31 does not shift from the longitudinal axis C in the width direction of the narrowing portion 31. It is to be noted that positions of the boundary positions E5 and E6 may be at a position equal to a distance (the same dimension) from the distal end Ed of the treatment portion 33 of the ultrasonic probe 8 along the longitudinal axis C to the boundary position E3, or may be at a position equal to a distance (the same dimension) from the distal end Ed along the longitudinal axis C to the boundary position E4.

Here, as described above, the boundary positions E3 and E4 shift forward and backward along the longitudinal axis C. In particular, the boundary position E3 of the first narrowing outer surface 41 is disposed closer to the proximal side than the boundary position E4 of the second narrowing outer surface 42. Furthermore, a dimension between the first relay surface 51 and the longitudinal axis C is larger than a dimension between the second relay surface 52 and the longitudinal axis C. A distance between the first relay surface 51 and the second relay surface 52, i.e., a thickness T of a distal portion of the narrowing portion 31 is from 1.55 mm to 1.65 mm in the certain example. In this case, a distance between the longitudinal axis C and the first relay surface 51 is from 0.95 mm to 1.1 mm, and the distance between the longitudinal axis C and the second relay surface 52 is from 0.45 mm to 0.7 mm. Consequently, the center of gravity of the distal portion of the narrowing portion 31 shifts to the first intersecting direction P1 side of the longitudinal axis C.

The sectional area of the narrowing portion 31 which is vertical to the longitudinal axis C decreases from the proximal side toward the distal side. In other words, in the treatment portion between the narrowing start position (the boundary position) E1 and the narrowing end position (the boundary position) E3 or E4 and between the narrowing start position (the boundary position) E2 and the narrowing end position (the boundary position) E5 or E6, the sectional area of the narrowing portion 31 which is vertical to the longitudinal axis C decreases from the proximal side toward the distal side.

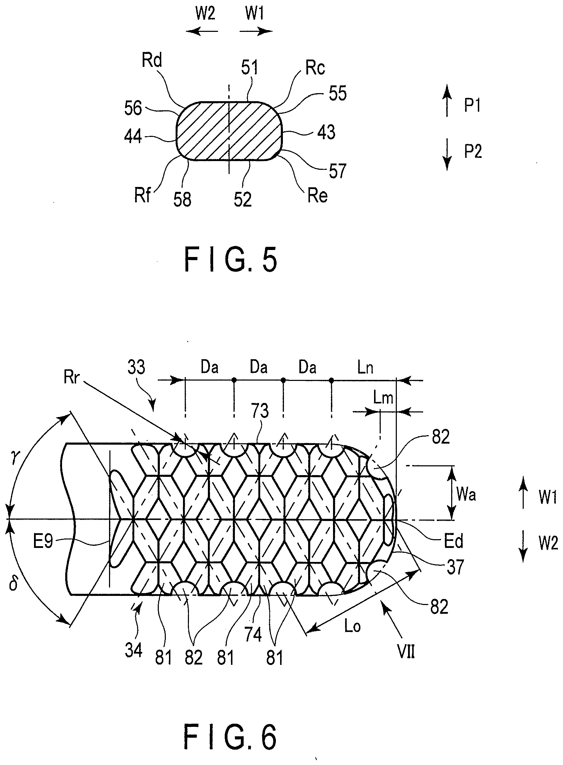

FIG. 5 is a cross-sectional view along the V-V line of FIG. 3, but drawing of the treatment portion 33 is omitted. FIG. 5 shows a sectional area vertical to the longitudinal axis C between the boundary position (the narrowing end position) E4 and a boundary position (a narrowing end position) E7 in the direction along the longitudinal axis C. As shown in FIG. 5, a curved surface (a first curved surface) 55 having a radius Rc is formed between the first relay surface 51 of the narrowing portion 31 and the third narrowing outer surface 43, and a curved surface (a second curved surface) 56 having a radius Rd is formed between the first relay surface 51 and the fourth narrowing outer surface 44. Furthermore, a curved surface (a third curved surface) 57 having a radius Re is formed between the second relay surface 52 of the narrowing portion 31 and the third narrowing outer surface 43, and a curved surface (a fourth curved surface) 58 having a radius Rf is formed between the second relay surface 52 and the fourth narrowing outer surface 44. In the certain example, each of the radiuses Rc and Rd is 0.75 mm and each of the radiuses Re and Rf is 0.5 mm.

Each of the curved surfaces 55 to 58 is not only formed between the boundary positions (the narrowing end positions) E4 and E7, but also extends in a range from the treatment portion 33 to the distal portion of the narrowing portion 31 in the direction along the longitudinal axis C. For example, each of the curved surfaces 55 and 56 extends in a range shown with a broken line B1 of FIG. 3 and each of the curved surfaces 57 and 58 extends in a range shown with a broken line B2 of FIG. 3. Therefore, in the distal portion of the narrowing portion 31, the bend extending portion 32 and the treatment portion 33, the curved surface 55 is formed between a region of an outer surface which is directed to the first intersecting direction P1 side and a region of the outer surface which is directed to one side (the arrow W1 side) in the width direction, and the curved surface 56 is formed between a region of the outer surface which is directed to the first intersecting direction side and a region of the outer surface which is directed to the other side (the arrow W2 side) in the width direction. Furthermore, in each of the distal portion (a relay extending portion) of the narrowing portion 31, the bend extending portion 32 and the treatment portion 33, the curved surface 57 is formed between a region of the outer surface which is directed to the second intersecting direction P2 side and a region of the outer surface which is directed to one side (the arrow W1 side) in the width direction, and the curved surface 58 is formed between a region of the outer surface which is directed to the second intersecting direction P2 side and a region of the outer surface which is directed to the other side (the arrow W2 side) in the width direction.

As shown in FIG. 2 to FIG. 4, distal ends of the first and second relay surfaces 51 and 52 and distal ends of the third and fourth narrowing outer surfaces 43 and 44 are positioned in the bend extending portion 32. The bend extending portion 32 includes a first extending surface 61 directed to the first intersecting direction side, a second extending surface 62 directed to the second intersecting direction side, a third extending surface 63 directed to the one side (the arrow W1 side) in the width direction, and a fourth extending surface 64 directed to the other side (the arrow W2 side) in the width direction. The first extending surface 61 is continuous with the distal side of the first relay surface 51 via the boundary position E7. The second extending surface 62 is continuous with the distal side of the second relay surface 52 via a boundary position E8. It is preferable that the first and second extending surfaces 61 and 62 are parallel to each other. The first extending surface 61 approaches or intersects the longitudinal axis C from the proximal side toward the distal side. The second extending surface 62 comes away from the longitudinal axis C from the proximal side toward the distal side. Furthermore, the cutting region 34 is provided on the side of the second extending surface 62.

The treatment portion 33 of the distal portion of the bend extending portion 32 includes a first continuous surface 71 continuous with the first extending surface 61, a second continuous surface 72 continuous with the second extending surface 62, the cutting region 34 directed to the second intersecting direction side (the arrow P2 side), a first extending end face 73 continuous with the third extending surface 63, and a second extending end face 74 continuous with the fourth extending surface 64. The first continuous surface 71 is directed to the first intersecting direction side (the arrow P1 side). The second continuous surface 72 is directed to the second intersecting direction side (the arrow P2 side). The cutting region 34 is directed to the second intersecting direction side (the arrow P2 side). Furthermore, the cutting region 34 is provided on the distal side of the second continuous surface 72. The first extending end face 73 is directed to the one side (the arrow W1 side shown in FIG. 4 and FIG. 6) in the width direction. The second extending end face 74 is directed to the other side (the arrow W2 side shown in FIG. 4 and FIG. 6) in the width direction. The continuous surface 71, the cutting region 34 and the first and second extending end faces 73 and 74 are continuous with the distal outer surface 37 on the distal side.

In addition, according to the present embodiment, a dimension W between the first extending end face 73 and the second extending end face 74 in the width direction is from 2.6 mm to 2.8 mm. Furthermore, in the present embodiment, the dimension W of the narrowing portion 31 in the width direction is from 2.6 mm to 2.8 mm at the narrowing end positions (the boundary positions) E5 and E6. It is to be noted that in this embodiment, the dimension W in the width direction is the same in regions on the distal side from the boundary positions E5 and E6 of the distal portion of the narrowing portion 31, i.e., the bend extending portion 32 and the treatment portion 33.

The boundary position E7 shown in FIG. 3 and FIG. 4 is determined by the distal end of the first relay surface 51 and a proximal end of the first extending surface 61. The first extending surface 61 approaches the longitudinal axis C from the boundary position E7 toward the distal side. The first extending surface 61 tilts at an angle α to the longitudinal axis C directed from the distal side toward the proximal side. Furthermore, the first continuous surface 71 of the treatment portion 33 which is continuous with the first extending surface 61 intersects the longitudinal axis C. The boundary position E8 in FIG. 3 and FIG. 4 is determined by the distal end of the second relay surface 52 and a proximal end of the second extending surface 62. The second extending surface 62 comes away from the longitudinal axis C from the boundary position E8 toward the distal side. The second extending surface 62 tilts at an angle β to the longitudinal axis C from the proximal side toward the distal side. A curved surface having a suitable radius Rg is formed at the boundary position E7 between the distal end of the first relay surface 51 and the proximal end of the first extending surface 61. A curved surface having a suitable radius Rh is formed at the boundary position E8 between the distal end of the second relay surface 52 and the proximal end of the second extending surface 62. In the certain example, each of the angles α and β is 7.5°. It is to be noted that a suitable distance Lg from the distal end Ed of the treatment portion 33 to the boundary position E7 is prescribed. A distance Lh from the distal end Ed of the treatment portion 33 to the boundary position E8 is from 7.5 mm to 8.5 mm in the certain example.

The boundary position E5 in FIG. 3 and FIG. 4 is determined by the distal end of the third narrowing outer surface 43 and a proximal end of the third extending surface 63. The boundary position E6 is determined by the distal end of the fourth narrowing outer surface 44 and a proximal end of the fourth extending surface 64. A curved surface having a suitable radius Ri is formed at the boundary position E5 between the distal end of the third narrowing outer surface 43 and the proximal end of the third extending surface 63. A curved surface having a suitable radius Rj is formed at the boundary position E6 between the distal end of the fourth narrowing outer surface 44 and the proximal end of the fourth extending surface 64.

A curved surface having a radius Rk is formed between the distal outer surface 37 of the treatment portion 33 and the first continuous surface 71. A curved surface having a radius Rl is formed between the distal outer surface 37 and the cutting region 34. A curved surface having a radius Rm is formed between the distal outer surface 37 and the first extending end face 73. A curved surface having a radius Rn is formed between the distal outer surface 37 and the second extending end face 74. Therefore, the distal outer surface 37 is continuous with each of the continuous surface 71, the first extending end face 73, the second extending end face 74 and the cutting region 34 of the treatment portion 33.

In the certain example, the radius Rk is 0.75 mm, the radius Rl is 0.5 mm, and each of the radiuses Rm and Rn is 1.25 mm. It is to be noted that in the present embodiment, the cutting region 34 is formed as a part of a spherical surface. A spherical surface radius SR of the cutting region 34 is 15 mm in the certain example.

The distal end of the second extending surface 62 determines a boundary position E9 (see FIG. 6) with the second continuous surface 72 of the treatment portion 33. In other words, a proximal end of a region of the treatment portion 33 on the second intersecting direction (the arrow P2 direction) side is determined by the boundary position E9. A curved surface 76 having a radius Ro is formed at the boundary position E9 between the distal end of the second extending surface 62 and a proximal end of the second continuous surface 72. The second continuous surface 72 continuous with a distal end of the second extending surface 62 via the curved surface 76 comes away toward the second intersecting direction (the arrow P2 direction) side of the longitudinal axis C. Consequently, due to the curved surface 76, a projecting amount of the second continuous surface 72 extending farther to the second intersecting direction side than the second extending surface 62 increases toward the distal side of the longitudinal axis C. The proximal end of the treatment portion 33 on the second intersecting direction (the arrow P2 direction) side is determined by the curved surface 76. A curved surface 77 having a radius Rp is formed on the distal side of the curved surface 76 along the longitudinal axis C. A distal end of the second continuous surface 72 is determined by the curved surface 77. The radius Rp of the curved surface 77 is continuous with the spherical surface radius SR of the cutting region 34. The curved surface 77 forms an edge of a proximal portion of the cutting region 34. In the certain example, the radius Ro is 0.75 mm and the radius Rp is 0.5 mm. A length of an operating region of the cutting region 34 that contributes to the cutting of the bone, the cartilage or the like is a distance Li from the distal end Ed of the treatment portion 33 to the edge of the proximal portion of the cutting region 34. The distance Li is 5 mm in the certain example.

A thickness T1 between a distal end of the first continuous surface 71 of the treatment portion 33 and the cutting region 34 is from 1.25 mm to 1.5 mm in the certain example. Here, when a cross section parallel to the longitudinal axis C and vertical to the first and second relay surfaces 51 and 52 is taken, the thickness T1 indicates a thickness of the cross section at a position where the cutting region 34 is farthest from the longitudinal axis C in a plane of the cross section. A distance Lj between the longitudinal axis C and the cutting region 34 is from 1.5 mm to 1.7 mm in the certain example. In the same manner as described above, the distance Lj indicates a distance at the position where the cutting region 34 is farthest from the longitudinal axis C in the plane of the cross section, when the cross section parallel to the longitudinal axis C and vertical to the first and second relay surfaces 51 and 52 is taken. Furthermore, a distance Lk between the distal end Ed of the treatment portion 33 and the position where the cutting region 34 is farthest from the longitudinal axis C is from 3 mm to 3.2 mm in the certain example.

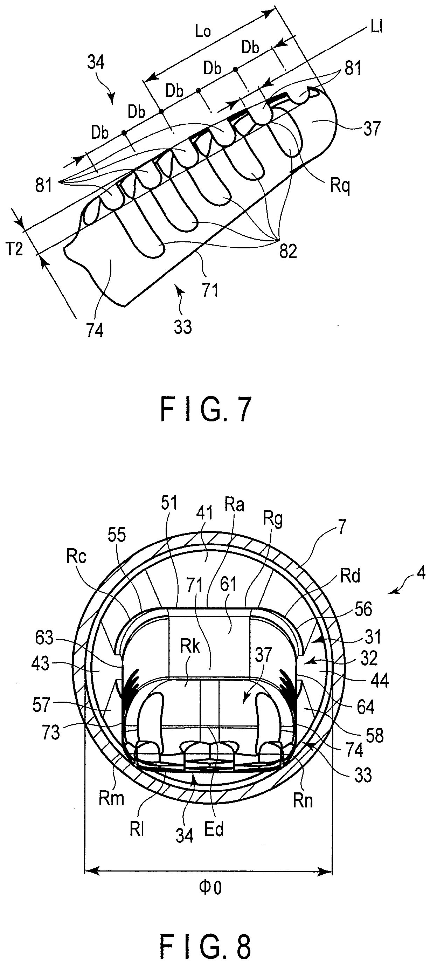

FIG. 6 and FIG. 7 are views showing a constitution of the treatment portion 33. FIG. 6 shows a state where the cutting region 34 of the treatment portion 33 is seen from the second intersecting direction (the arrow P2) side, and FIG. 7 shows a state where the treatment portion 33 is seen from a direction of an arrow VII of FIG. 6.

As shown in FIG. 6, the cutting region 34 is formed into a reticulated crosshatch pattern that is tilted to the longitudinal axis C. Grooves 81 are crossed in the cutting region 34. Each groove 81 is straightly formed in the present embodiment. As shown in FIG. 7, a distance L1 between edges of the grooves 81 is 0.4 mm in the certain example. Furthermore, the edges of the respective grooves 81 contribute to the cutting of the bone, the cartilage or the like. Each of tilt angles γ and δ of the respective grooves 81 to the longitudinal axis C from the distal side toward the proximal side shown in FIG. 6 is 60° in the certain example. A radius Rq of each groove 81 in a depth direction is 0.2 mm in the certain example.

A concave portion 82 having a radius Rr is formed every suitable interval Da along the longitudinal axis C in each of the first and second extending end faces 73 and 74 of the cutting region 34 in the width direction. The concave portions 82 are continuous with the grooves 81. For example, two grooves 81 are continuous with a certain concave portion 82 of the first extending end face 73. Similarly, two grooves 81 are continuous with a certain concave portion 82 of the second extending end face 74.

In the certain example, the radius Rr is, for example, 0.25 mm. The interval Da between centers of the respective concave portions 82 along the longitudinal axis C is 0.9 mm in the certain example.

The concave portions 82 are also formed in the distal outer surface 37 mentioned above. The distal end Ed of the distal outer surface 37 of the treatment portion 33 is disposed away from the concave portion 82 of the distal outer surface 37 as much as a width Wa in the width direction to the longitudinal axis C and as much as a distance Lm in an axial direction. In the certain example, the width Wa is 1 mm and the distance Lm is 0.3 mm. A distance Ln between the distal end Ed of the distal outer surface 37 and the most distal concave portion 82 along the longitudinal axis C in each of the first and second extending end faces 73 and 74 which are continuous with the distal outer surface 37 is 1.2 mm in the certain example.

As shown in FIG. 7, a depth T2 of the groove 81 is about 0.5 mm at maximum. Furthermore, the respective grooves 81 are disposed away from each other at an interval Db of 0.8 mm in the certain example as seen from a direction of the angles γ and δ to the longitudinal axis C. A distance Lo between the distal end Ed of the distal outer surface 37 and the groove 81 continuous with the third concave portion 82 from the distal end Ed on the proximal side is 2.85 mm in the certain example.

FIG. 8 is a view of the sheath 7 and the ultrasonic probe 8 which are seen from the distal side. As shown in FIG. 8, the sheath 7 has a minimum inner diameter φo. The minimum inner diameter φo of the sheath 7 is larger than the outer diameter φa of the ultrasonic probe 8 at the boundary position E1 between the probe main body 15 and the narrowing portion 31. In an example where the outer diameter φa is 3.8 mm, the minimum inner diameter φo of the sheath 7 is 4 mm. In an example where the outer diameter φa is 2.9 mm, the minimum inner diameter φo of the sheath 7 is 3.4 mm. In projection seen from the distal side, the narrowing portion 31, the bend extending portion 32 and the treatment portion 33 are arranged in a range inner than the minimum inner diameter φo of the sheath 7.

Next, operations and effects of the ultrasonic probe 8 and the ultrasonic treatment instrument 2 of the present embodiment will be described. The ultrasonic treatment system 1 is for use in a treatment of cutting the bone, the cartilage or the like in a joint such as the knee joint, the shoulder joint, the elbow joint or the like under a viewing field of an unshown arthroscope. The ultrasonic probe 8 of the present embodiment is also usable in the shoulder joint, but is preferably for use in a treatment of a comparatively narrow joint such as the knee joint, the elbow joint or the like.

In the treatment, the distal portion of the ultrasonic probe 8 and a distal portion of the sheath 7 shown in FIG. 1 are inserted into a joint cavity of a joint J through a port (not shown) formed by a cannula or the like. Then, the cutting region 34 of the treatment portion 33 is brought into contact with a treatment target (e.g., an affected part formed in the bone, the cartilage or the like) Ap in the joint cavity. Then, in a state where the cutting region 34 is in contact with the treatment target Ap, the operator performs operation input with the operation button 9. Consequently, the ultrasonic vibration is generated in the ultrasonic transducer 12 shown in FIG. 2, and the ultrasonic vibration generated in the vibrating body unit 10 is transmitted from the proximal side to the distal side. In a state where the ultrasonic vibration is being transmitted, the vibrating body unit 10 performs longitudinal vibration in a vibrating direction substantially parallel to the longitudinal axis C. Thus, the treatment portion 33 longitudinally vibrates along the longitudinal axis C in the state where the cutting region 34 is in contact with the treatment target Ap, whereby the treatment target (the bone, the cartilage or the like) is cut.

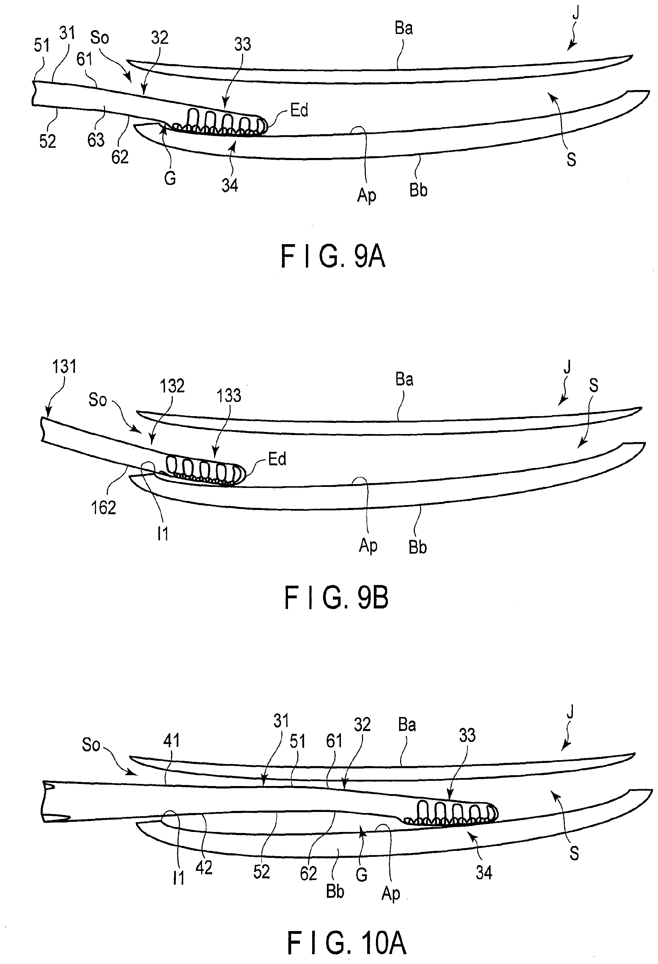

FIG. 9A and FIG. 10A are views showing one example of a state of cutting the treatment target Ap in the joint cavity of the joint J with the cutting region 34. As shown in FIG. 9A and FIG. 10A, it is required to cut the treatment target Ap in a narrow space of the joint cavity. For example, there is a case of cutting the affected part Ap that is the treatment target in the narrow space denoted with sign S between a bone Ba and a bone Bb. It is necessary to bring the cutting region 34 into contact with the treatment target Ap in the narrow space S, and hence an angle range of an intruding angle (i.e., an approach angle of the cutting region 34 to the treatment target Ap) of the cutting region 34 when the cutting region 34 approaches the treatment target Ap is limited to a small range.

Here is described a shape of a range (a range shown with the broken line B2 in FIG. 3) of the second narrowing outer surface 42, the second relay surface 52 and the second extending surface 62 on the second intersecting direction P2 side in the ultrasonic probe 8. In the second narrowing outer surface 42 of the narrowing portion 31, a distance (a first distance) D1 on the second intersecting direction P2 side which is perpendicular to the straight longitudinal axis C increases from the distal side toward the proximal side along the longitudinal axis C. In a region between the distal end of the second relay surface 52 of the narrowing portion 31 (the boundary position E8) and the proximal end thereof (the boundary position E4), a distance (a second distance) D2 to the longitudinal axis C is smaller than the distance D1 and uniform. The distance D2 to the longitudinal axis C is also uniform at the distal end of the second narrowing outer surface 42 (the boundary position E4) and the proximal end of the second extending surface 62 (the boundary position E8). In the second extending surface 62 of the bend extending portion 32, a distance D3 (> D2) to the longitudinal axis C on the second intersecting direction P2 side increases toward the distal side. Furthermore, the distance Lj between the longitudinal axis C and the cutting region 34 is larger than the distance (a third distance) D3 at any position. Consequently, as described above, the cutting region 34 is provided at a position more away from the longitudinal axis C than the bend extending portion 32 in the second intersecting direction P2 from the longitudinal axis C. Therefore, according to the ultrasonic probe 8 of this embodiment, in the state where the cutting region 34 is in contact with the treatment target Ap, it is easy to form a gap (space) G (see FIG. 9A and FIG. 10A) between a tissue or the like adjacent to the proximal side of the cutting region 34 and each of the second relay surface 52 and the second extending surface 62. The gap G can contribute to inhibition of interference with the tissue or the like adjacent to the proximal side of the cutting region 34.

FIG. 9A and FIG. 10A show a state where the treatment portion 33 of the ultrasonic probe 8 is brought into contact with the affected part Ap in the narrow space S of the joint cavity between the upper bone Ba and the lower bone Bb. In the probe 8 of the present embodiment, as described above, the distal side of the narrowing portion 31 is bent in the bend extending portion 32 on the second intersecting direction P2 side, and the probe 8 has the cutting region 34 in the bent portion on the second intersecting direction P2 side. In other words, on the proximal side of the cutting region 34, the gap (space) G is formed by the second extending surface 62 of the bend extending portion 32 and the second relay surface 52 of the narrowing portion 31. Consequently, when the cutting region 34 of the treatment portion 33 is brought into contact with the affected part Ap from an opening So of the narrow space S of the joint cavity on a left side of FIG. 9A, a position in the vicinity of the opening So of the narrow space S of the joint cavity is hard to interfere with the second extending surface 62. Furthermore, as shown in FIG. 10A, also when the cutting region 34 of the treatment portion 33 is brought into contact with the affected part Ap at a position inner than the position shown in FIG. 9A from the opening So of the narrow space S of the joint cavity on a left side of FIG. 10A, the position in the vicinity of the opening So of the narrow space S of the joint cavity is hard to interfere with the narrowing portion 31 and the bend extending portion 32.

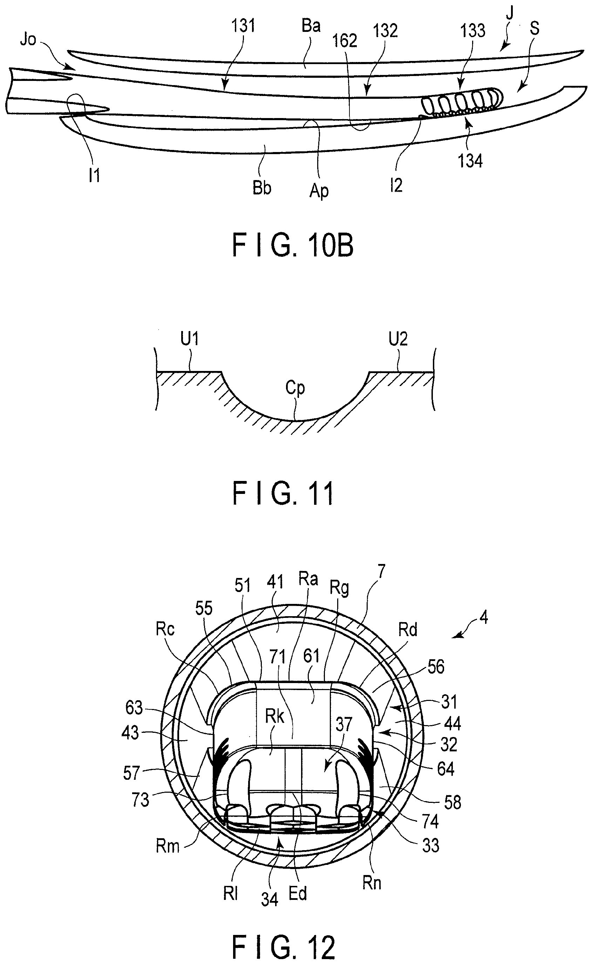

FIG. 9B and FIG. 10B show, as a reference, an example where a shape of a distal portion of a narrowing portion 131 to a treatment portion 133 is different from the present embodiment. A bend extending portion 132 is bent from the distal portion of the narrowing portion 131 of a probe 108 of this reference example to a first intersecting direction P1 side opposite to the side of the present embodiment. Consequently, when a cutting region 134 of the treatment portion 133 is brought into contact with an affected part Ap from an opening So of a narrow space S of a joint cavity on a left side of FIG. 9B, the opening So of the narrow space S of the joint cavity which is an interfering area I1 is likely to interfere with a second extending surface 162 of the bend extending portion 132. Furthermore, as shown in FIG. 10B, also when the cutting region 134 of the treatment portion 133 is brought into contact with the affected part Ap at a position inner than the position shown in FIG. 9B from the opening So of the narrow space S of the joint cavity on a left side of FIG. 10B, the opening So of the narrow space S of the joint cavity which is the interfering area I1 is likely to interfere with the second extending surface 162 of the bend extending portion 132, and a position denoted with sign I2 as an interfering region in a proximal portion of the cutting region 134 is likely to interfere with the affected part Ap. In other words, in the example shown as the reference in FIG. 9B and FIG. 10B, the gap G from the treatment target shown in FIG. 9A and FIG. 10A is not formed.

Thus, in the ultrasonic probe 8 according to the present embodiment, the bend extending portion 32 is provided on the distal side of the narrowing portion 31. The bend extending portion 32 extends in the state of bending to the second intersecting direction P2 side of the longitudinal axis C. Furthermore, in the bend extending portion 32, the cutting region 34 is formed toward the second intersecting direction P2 side. Due to the above-mentioned constitution, the cutting region 34 can first come in contact with the affected part Ap even in the narrow space S of the joint cavity in which the angle range of the approach angle to the treatment target Ap is limited to a small range, and hence a region of the ultrasonic probe 8 excluding the cutting region 34 is prevented from interfering with the tissue or the like other than the treatment target Ap (e.g., an area other than the affected part Ap in the bone Ba) . Consequently, a position of the treatment target Ap excluding an area in contact with the cutting region 34 is hard to come in contact with the ultrasonic probe 8, and hence unintended cutting by the ultrasonic vibration can be inhibited. In consequence, also in the narrow space S, the cutting region 34 that is a blade region appropriately comes in contact with the treatment target Ap, a treatment performance in the treatment of cutting the treatment target Ap is acquired, and the treatment can efficiently be performed.

Furthermore, in the present embodiment, as shown in FIG. 8, the narrowing portion 31, the bend extending portion 32 and the treatment portion 33 are arranged in the range inner than the minimum inner diameter φo of the sheath 7 in the projection seen from the distal side. Consequently, in the narrow space S of the joint cavity, the region other than the cutting region 34 in the ultrasonic probe 8 is further effectively prevented from interfering with the tissue or the like other than the treatment target Ap. Consequently, in the narrow space S, the cutting region 34 that is the blade region further appropriately comes in contact with the treatment target Ap.

The present embodiment has a constitution where the narrowing portion 31, the bend extending portion 32 and the treatment portion 33 are arranged in the range inner than the minimum inner diameter φo of the sheath 7. Consequently, it is easy to insert the ultrasonic probe 8 through the sheath 7. In consequence, time and labor in assembling the ultrasonic treatment instrument 2 are decreased.

FIG. 11 is a view showing the treatment target cut with the cutting region 34. As described above, the cutting region 34 is formed as a part of the substantially spherical surface having the radius SR. Consequently, in the present embodiment, as shown in FIG. 11, edges having acute angles are not formed between a cut plane Cp obtained by removing the treatment target and each of uncut planes U1 and U2 adjacent to the cut plane Cp in the bone, the cartilage or the like. Furthermore, the cutting region 34 is formed as a part of the spherical surface, and hence the cut plane Cp from which the treatment target is removed has a dented area having a substantially circular cross section.

Furthermore, in the present embodiment, the vicinity of the distal portion of the narrowing portion 31 (a region where the first and second relay surfaces 51 and 52 and the third and fourth narrowing outer surfaces 43 and 44 form a ring-shaped outer peripheral surface) shifts to the first intersecting direction P1 side of the longitudinal axis C. On the other hand, the bend extending portion 32 extends in the state of bending to the second intersecting direction P2 side of the longitudinal axis C. Consequently, the vicinity of the distal portion of the narrowing portion 31 and the center of gravity in the whole bend extending portion 32 including the treatment portion 33 do not noticeably shift from the longitudinal axis C in the first intersecting direction P1 and the second intersecting direction P2. Therefore, in the ultrasonic probe 8 according to the present embodiment, there is inhibited transverse vibration (improper vibration) in a vibrating direction substantially parallel to the first intersecting direction P1 and the second intersecting direction P2.

In this embodiment, it has been described that the second relay surface 52 is parallel to the longitudinal axis C (the distance D2 is uniform) as shown in FIG. 3, but the second relay surface does not have to be parallel. Also in this case, it is preferable that a relation of the distance D1 > the distance D2 is established. Furthermore, at the boundary position E4, the distance D1 matches the distance D2. Furthermore, needless to say, the distance D2 may be adjusted to change in accordance with a position along the longitudinal axis C as in the distances D1 and D3.

It is to be noted that in the ultrasonic probe 8 according to the present embodiment, the width directions W1 and W2 are set symmetrically to the longitudinal axis C. Consequently, there is inhibited the transverse vibration (the improper vibration) in the vibrating direction substantially parallel to the width directions W1 and W2.

The treatment portion 33 of the ultrasonic probe 8 to the sheath 7 is not limited to the example shown in FIG. 8, and it is also preferable that the treatment portion is formed as in a modification shown in FIG. 12.

FIG. 12 shows a state where in projection seen from the distal side of the longitudinal axis C, the narrowing portion 31, the bend extending portion 32 and the treatment portion 33 are arranged in a range inner than the outer diameter φa of the ultrasonic probe 8 at the boundary position E1. Furthermore, the narrowing portion 31, the bend extending portion 32 and the treatment portion 33 are capable of performing the treatment as described in the first embodiment, even in the state where the portions are arranged in the range inner than the outer diameter φa of the ultrasonic probe 8 at the boundary position E1 as shown in FIG. 12. Therefore, as long as the portions are arranged in a range inner than the inner diameter φo of the sheath 7, the treatment portion 33 may be in contact with the outer diameter φa of the ultrasonic probe 8.

### (Second Embodiment)

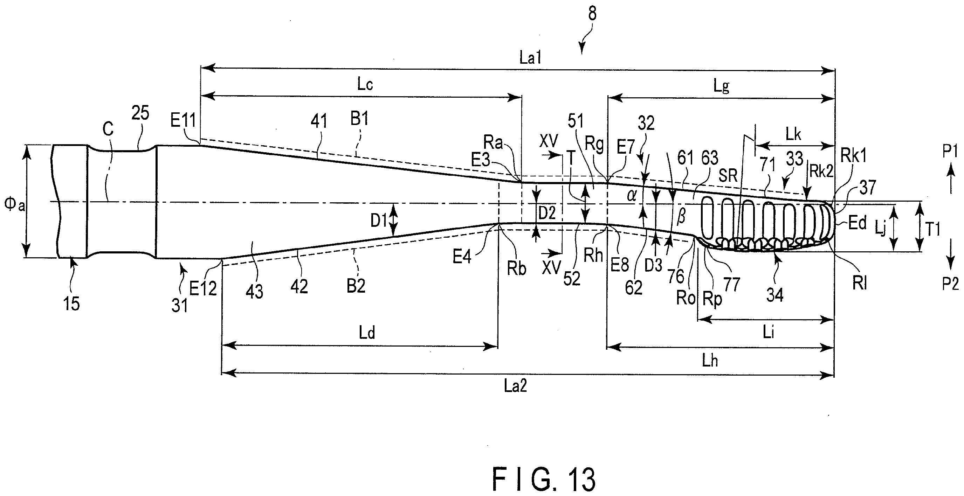

Next, a second embodiment will be described with reference to FIG. 13 to FIG. 17. This embodiment is a modification of the first embodiment, the same members or members having the same functions as in the members described in the first embodiment are denoted with the same signs to the utmost, and detailed description is omitted.

An ultrasonic probe 8 according to this embodiment has an outer diameter φa larger than that described in the first embodiment, and is preferably for use mainly in a treatment of a shoulder joint. The ultrasonic probe 8 according to this embodiment has the outer diameter φa of, for example, 4.9 mm.

The ultrasonic probe 8 of the present embodiment has about the same shape as in the ultrasonic probe 8 described in the first embodiment, and is suitably different in dimension. Consequently, different portions between both the embodiments will mainly be described.

As shown in FIG. 13, first and second extending surfaces 61 and 62 are not parallel to each other in this embodiment. In a certain example, an angle α is 5° and an angle β is 7.5°.

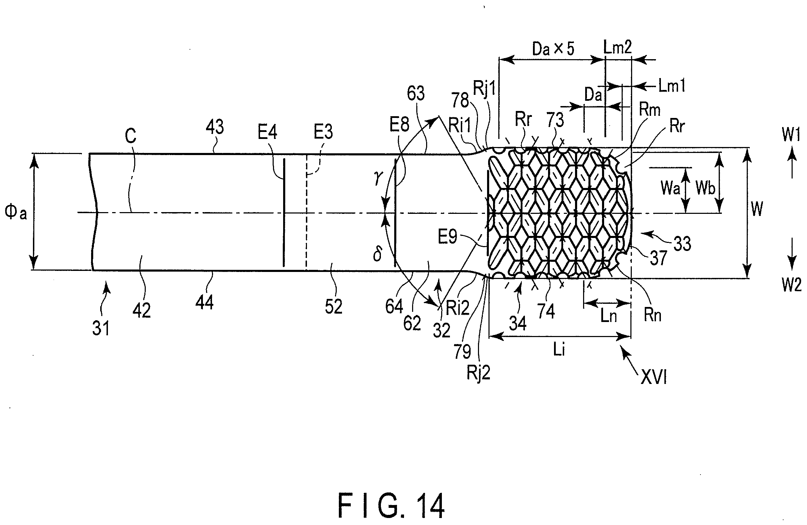

As shown in FIG. 14, in a narrowing portion 31, third and fourth narrowing outer surfaces 43 and 44 are parallel to each other. Consequently, a boundary position E2 prescribed in the first embodiment is not prescribed in this embodiment. In this embodiment, a boundary position E5 is not present between the third narrowing outer surface 43 and a third extending surface 63. Similarly, a boundary position E6 is not present between the fourth narrowing outer surface 44 and a fourth extending surface 64. Consequently, the third narrowing outer surface 43 and the third extending surface 63 are parallel to the fourth narrowing outer surface 44 and the fourth extending surface 64, respectively. Therefore, here, radiuses Ri and Rj (see FIG. 4) are not prescribed.

In a region between a bend extending portion 32 and a treatment portion 33, the third extending surface 63 is continuous with a first extending end face 73 on a distal side of the third extending surface via a curved surface 78. The fourth extending surface 64 is continuous with a second extending end face 74 on a distal side of the fourth extending surface via a curved surface 79. Consequently, a dimension W in a width direction is uniform in the treatment portion 33, but in this embodiment, the dimension is not the same in a region on the distal side from boundary positions E3 and E4 of a distal portion of the narrowing portion 31, i.e., in the bend extending portion 32 and the treatment portion 33.

In the curved surface 78 between a distal end of the third extending surface 63 and a proximal end of the first extending end face 73, suitable radiuses Ri1 and Rj1 are continuous from a proximal side toward the distal side. Due to the radius Ri1, a distance of the first extending end face 73 from a longitudinal axis C is larger than that of the third extending surface 63. The proximal end of the first extending end face 73 is determined with the radius Rj1. Similarly, in the curved surface 79 between a distal end of the fourth extending surface 64 and a proximal end of the second extending end face 74, suitable radiuses Ri2 and Rj2 are continuous from the proximal side toward the distal side. Due to the radius Ri2, a distance of the second extending end face 74 from the longitudinal axis C is larger than that of the fourth extending surface 64. The proximal end of the second extending end face 74 is determined with the radius Rj2.

Here, the boundary position E1 described in the first embodiment is not the same as in first and second narrowing outer surfaces 41 and 42, and separate boundary positions E11 and E12 are prescribed, respectively. In the certain example, a dimension La1 from a distal end Ed of the treatment portion 33 of the ultrasonic probe 8 to the boundary position E11 between a probe main body 15 and the narrowing portion 31 on the side of the first narrowing outer surface 41 in a direction along the longitudinal axis C (a longitudinal direction) is 27.8 mm. A dimension La2 from the distal end Ed of the treatment portion 33 of the ultrasonic probe 8 to the boundary position E12 between the probe main body 15 and the narrowing portion 31 on the side of the second narrowing outer surface 42 in the direction along the longitudinal axis C (the longitudinal direction) is 26.8 mm. In other words, the boundary positions E11 and E12 may shift along the longitudinal axis C on the sides of the first and second narrowing outer surfaces 41 and 42.

The dimension Lb prescribed in the first embodiment is not prescribed here. This is because in the present embodiment, a distance from the longitudinal axis C to an outer surface directed in the width direction is uniform from a distal end of a supported portion 25 to the bend extending portion 32. Furthermore, dimensions Le and Lf are not prescribed similarly.

It is to be noted that a dimension Lc is 14 mm and a dimension Ld is 12 mm. Each of distances Lg and Lh is 10 mm, a distance Li is 6 mm, a distance Lj is 2.05 mm, a distance Lk is 3.5 mm, and a distance Ll is 0.5 mm. A distance Ln is 2 mm and a distance Lo is 3.7 mm.

Here, the distal end Ed of a distal outer surface 37 of the treatment portion 33 is disposed away from concave portions 82 of the distal outer surface 37 as much as widths Wa and Wb in the width direction to the longitudinal axis C and as much as distances Lm1 and Lm2 in an axial direction. The width Wa is 1.9 mm, the width Wb is 2.55 mm, the distance Lm1 is 0.4 mm, and the distance Lm2 is 1.1 mm.

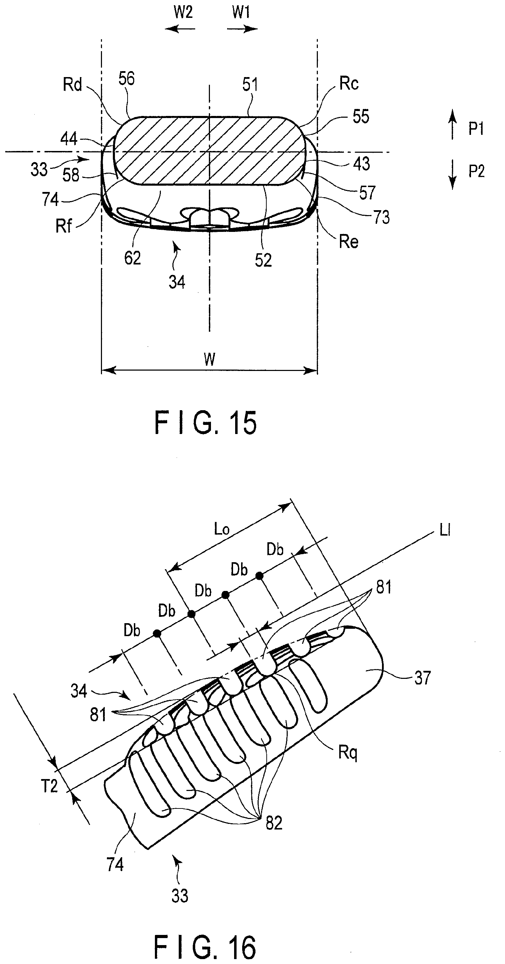

FIG. 15 is a cross-sectional view along the XV-XV line of FIG. 13. FIG. 15 shows a cross section vertical to the longitudinal axis C between the boundary position (a narrowing end position) E4 and a boundary position E7 in a direction along the longitudinal axis C or between the boundary position (the narrowing end position) E4 and a boundary position E8. In the certain example, each of radiuses Rc, Rd, Re and Rf is 0.75 mm.

Furthermore, curved surfaces having radiuses Rk1 and Rk2 are formed between the distal outer surface 37 of the treatment portion 33 and a first continuous surface 71. The radius Rk1 is 0.75 mm and the radius Rk2 is 15 mm.

A radius R1 is 0.75 mm, each of radiuses Rm and Rn is 2 mm, a radius Ro is 0.3 mm, a radius Rp is 0.75 mm, a radius Rq is 0.25 mm, a radius Rr is 0.25 mm, and a radius SR is 12.5 mm.

A thickness T1 between a distal end of the first continuous surface 71 and a cutting region 34 is 2.2 mm in the certain example.

A distance between a first relay surface 51 and a second relay surface 52, i.e., a thickness T is 1.75 mm in the certain example. In this case, a distance between the longitudinal axis C and the first relay surface 51 is 0.9 mm and a distance D2 between the longitudinal axis C and the second relay surface 52 is 0.85 mm. Consequently, a center of gravity of the distal portion of the narrowing portion 31 shifts to a first intersecting direction P1 side of the longitudinal axis C in the same manner as described in the first embodiment.

The dimension W in the width direction is 5.5 mm in the treatment portion 33. On the other hand, a dimension of each of the narrowing portion 31 and the bend extending portion 32 in the width direction is the same as an outer diameter φa.

As shown in FIG. 16, it is preferable that each groove 81 is straightly formed also in the present embodiment. In the certain example, the radius Rr of the concave portion 82 is 0.25 mm, and an interval Da between centers of the concave portions 82 along the longitudinal axis C is 0.9 mm. The radius Rq of each groove 81 in a depth direction is 0.25 mm in the certain example. In the certain example, a depth T2 of the groove 81 is 0.6 mm. Furthermore, the respective grooves 81 are disposed away from each other at an interval Db of 1 mm in the certain example seen from a direction of angles γ and δ to the longitudinal axis C.

Here is described a shape of a range (a range shown with a broken line B2 in FIG. 13) of the second narrowing outer surface 42, the second relay surface 52 and the second extending surface 62 on a second intersecting direction P2 side in the ultrasonic probe 8. In the second narrowing outer surface 42 of the narrowing portion 31, a distance (a first distance) D1 on the second intersecting direction P2 side which is perpendicular to the straight longitudinal axis C increases from the distal side toward the proximal side along the longitudinal axis C. In a region between a distal end of the second relay surface 52 of the narrowing portion 31 (the boundary position E8) and a proximal end thereof (the boundary position E4), the distance (a second distance) D2 to the longitudinal axis C is smaller than the distance D1 and uniform. The distance D2 to the longitudinal axis C is also uniform at a distal end of the second narrowing outer surface 42 (the boundary position E4) and a proximal end of the second extending surface 62 (the boundary position E8). In the second extending surface 62 of the bend extending portion 32, a distance D3 (> D2) to the longitudinal axis C on the second intersecting direction P2 side increases toward the distal side. Furthermore, the distance Lj between the longitudinal axis C and the cutting region 34 is larger than the distance D3 at any position. Consequently, as described above, the cutting region 34 is provided at a position more away from the longitudinal axis C than the bend extending portion 32 in the second intersecting direction P2. Therefore, according to the ultrasonic probe 8 of this embodiment, in the state where the cutting region 34 is in contact with a treatment target Ap, it is easy to form a gap (space) G (see FIG. 9A and FIG. 10A) between a tissue or the like adjacent to the proximal side of the cutting region 34 and each of the second relay surface 52 and the second extending surface 62. The gap G can contribute to inhibition of interference with the tissue or the like adjacent to the proximal side of the cutting region 34.

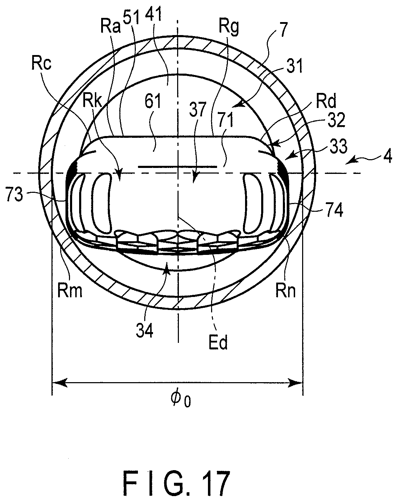

FIG. 17 is a view of a sheath 7 and the ultrasonic probe 8 from the distal side. A minimum inner diameter φo of the sheath 7 is larger than the outer diameter φa of the ultrasonic probe 8 at the boundary position E11 between the probe main body 15 and the narrowing portion 31. In projection seen from the distal side, the narrowing portion 31 and the bend extending portion 32 are arranged in a range inner than the minimum inner diameter φo of the sheath 7.

### (Third Embodiment)

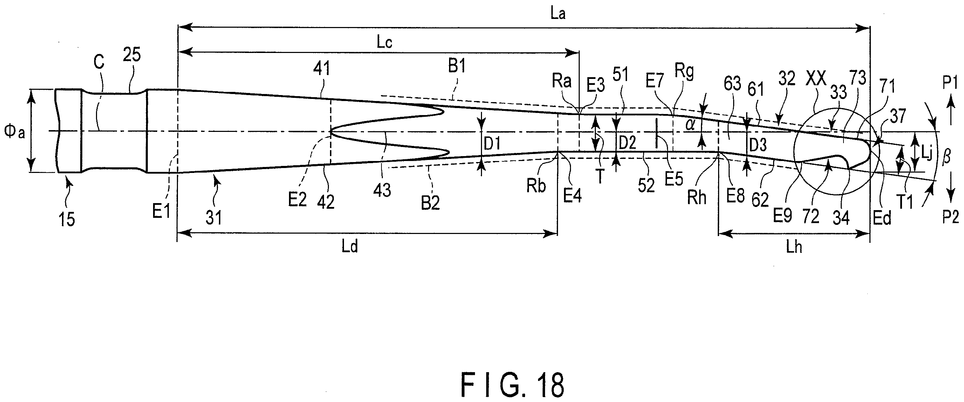

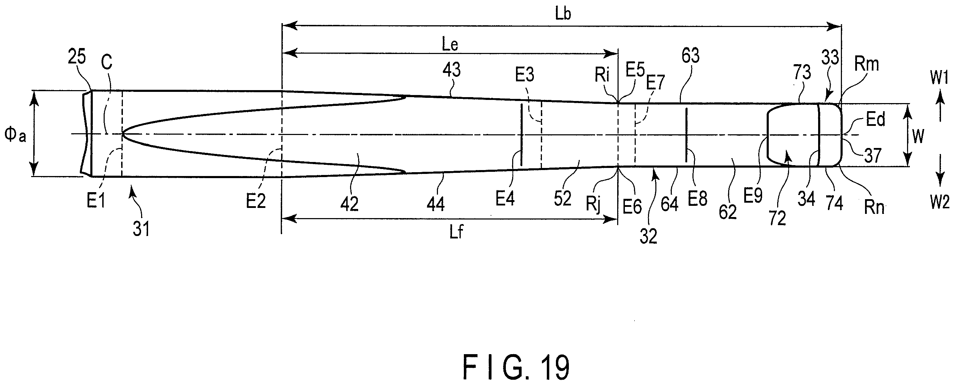

Next, a third embodiment will be described with reference to FIG. 18 to FIG. 21. This embodiment is a modification of the first and second embodiments, the same members or members having the same functions as in the members described in the first and second embodiments are denoted with the same signs to the utmost, and detailed description is omitted.

As shown in FIG. 18 to FIG. 21, a treatment portion 33 of a probe 8 according to this embodiment is formed into a rake shape (a hook shape). Similarly to the probe described in the first and second embodiments, also in the probe 8 according to this embodiment, a bend extending portion 32 is present on a distal side of a narrowing portion 31 and the treatment portion 33 is present on a distal side of the bend extending portion 32.

Here, a distance between a first relay surface 51 and a second relay surface 52, i.e., a thickness T of a distal portion of the narrowing portion 31 is 1.7 mm in a certain example. In this case, a distance between a longitudinal axis C and the first relay surface 51 is 0.8 mm and a distance D2 between the longitudinal axis C and the second relay surface 52 is 0.9 mm. Consequently, a center of gravity of the distal portion of the narrowing portion 31 to a second intersecting direction P2 side of the longitudinal axis C in this embodiment.

Here, on the distal side of the narrowing portion 31, due to the bend extending portion 32 bent to the second intersecting direction P2 side, a first extending surface 61 tilts at an angle α to the longitudinal axis C from the distal side toward a proximal side, and a second extending surface 62 tilts at an angle β to the longitudinal axis C from the proximal side toward the distal side. The angles α and β are the same in the certain example. Consequently, the first and second extending surfaces 61 and 62 are parallel to each other. It is to be noted that in the certain example, each of the angles α and P is 7.5°.

A first continuous surface 71 of the treatment portion 33 which is continuous with the first extending surface 61 of this embodiment also intersects the longitudinal axis C due to the bend extending portion 32 bent to the second intersecting direction P2 side on the distal side of the narrowing portion 31. Here is described an example where the first continuous surface 71 intersects the longitudinal axis C, but the first continuous surface may only approach the longitudinal axis C from the proximal side toward the distal side due to the bend extending portion 32 on the distal side of the narrowing portion 31.

Furthermore, a cutting region 34 of the treatment portion 33 is disposed on the second intersecting direction P2 side of the longitudinal axis C due to the bend extending portion 32 in the same manner as described in the first and second embodiments.

A second continuous surface 72 is formed on the distal side of the second extending surface 62. A boundary position E9 is formed between a distal end of the second extending surface 62 and the second continuous surface 72. Here, in the second continuous surface 72, a first flat portion 72a, a curved portion 72b and a second flat portion 72c are continuous from the proximal side toward the distal side.

The first flat portion 72a forms an edge at the boundary position E9 between the distal end of the second extending surface 62 and the second continuous surface. The distal side of the first flat portion 72a is disposed closer to the longitudinal axis C than the boundary position E9. In the curved portion 72b, the distal side of the first flat portion 72a is disposed away from the longitudinal axis C, from the proximal side toward the distal side. The curved portion 72b is formed to have a radius Rp. On the distal side of the curved portion 72b, the second flat portion 72c is formed continuously with the curved portion 72b. In the certain example, the radius Rp is 0.5 mm.

On the distal side of the second flat portion 72c, a flat portion 75 is formed to approach the longitudinal axis C from the proximal side toward the distal side. The cutting region 34 is formed into an edge between the second flat portion 72c and the flat portion 75. In other words, according to this embodiment, a spherical cutting region 34 (see FIG. 3) described in the first and second embodiments is not used.

It is preferable that an angle θ between the second flat portion 72c and the flat portion 75 is smaller than 90°. It is preferable that the cutting region 34 extends in a direction perpendicular to the longitudinal axis C and extends in parallel with a width direction.

It is to be noted that here, the cutting region 34 is present at a position to come in contact with the second extending surface 62, when the second extending surface is a virtually extending plane. In other words, at least a part of the cutting region 34 is present on the virtually extending plane of the second extending surface 62.

A height H1 of the cutting region 34 to the first flat portion 72a is, for example, 0.7 mm. A height H2 (a dimension Lk) of the cutting region 34 to a distal end Ed of the treatment portion 33 along the longitudinal axis C is, for example, 1 mm. An angle ε formed between the first flat portion 72a and the second flat portion 72c is 90° in the certain example. An angle ζ formed between the second flat portion 72c and the first continuous surface 71 is 72.5° in the certain example. An angle η formed between the first continuous surface 71 and the flat portion 75 is 30° in the certain example.

It is to be noted that in the certain example, a dimension La from the distal end Ed of the treatment portion 33 of the ultrasonic probe 8 to a boundary position E1 between a probe main body 15 and the narrowing portion 31 on a first narrowing outer surface 41 side and a second narrowing outer surface 42 side in a direction along the longitudinal axis C (a longitudinal direction) is 32 mm. A dimension Lb is 25 mm, a dimension Lc is 18.5 mm, and a dimension Ld is 17.5 mm. Each of dimensions Le and Lf is 15 mm. Here, a distance Lh is 7 mm and smaller than a distance Lg. A distance Li from the distal end Ed of the treatment portion 33 to an edge of a proximal portion of the cutting region 34 is not prescribed. A distance Lj is 1.7 mm.

Although not shown in the drawing, each of radiuses Rc, Rd, Re and Rf of curved surfaces 55, 56, 57 and 58 of a transverse cross section in the bend extending portion 32 formed similarly to FIG. 5 is 0.5 mm. Each of radiuses Rk and Rl is 0.5 mm.

Here is described a shape of a range (a range shown with a broken line B2 in FIG. 18) of the second narrowing outer surface 42, the second relay surface 52 and the second extending surface 62 on the second intersecting direction P2 side in the ultrasonic probe 8. In the second narrowing outer surface 42 of the narrowing portion 31, a distance (a first distance) D1 on the second intersecting direction P2 side which is perpendicular to the straight longitudinal axis C increases from the distal side toward the proximal side along the longitudinal axis C. In a region between a distal end of the second relay surface 52 of the narrowing portion 31 (a boundary position E8) and a proximal end thereof (a boundary position E4), the distance (a second distance) D2 to the longitudinal axis C is smaller than the distance D1 and uniform. The distance D2 to the longitudinal axis C is also uniform at a distal end of the second narrowing outer surface 42 (the boundary position E4) and a proximal end of the second extending surface 62 (the boundary position E8). In the second extending surface 62 of the bend extending portion 32, a distance D3 (> D2) to the longitudinal axis C on the second intersecting direction P2 side increases toward the distal side. Furthermore, the distance Lj between the longitudinal axis C and the cutting region 34 is larger than the distance D3 at any position. Consequently, as described above, the cutting region 34 is provided at a position more away from the longitudinal axis C than the bend extending portion 32 in the second intersecting direction P2. Therefore, according to the ultrasonic probe 8 of this embodiment, in the state where the cutting region 34 is in contact with a treatment target Ap, it is easy to form a gap (space) G (see FIG. 9A and FIG. 10A) between a tissue or the like adjacent to the proximal side of the cutting region 34 and each of the second relay surface 52 and the second extending surface 62. The gap G can contribute to inhibition of interference with the tissue or the like adjacent to the proximal side of the cutting region 34.

The probe 8 of this embodiment is likely to form the gap (space) G (see FIG. 9A and FIG. 10A) especially between the second relay surface 52 and the tissue or the like other than the treatment target during a treatment. Consequently, in the same manner as described in the first embodiment, the interference with the tissue or the like other than the treatment target is inhibited by use of the probe 8 of this embodiment. Consequently, the ultrasonic probe 8 is hard to come in contact with a position of the treatment target Ap excluding an area in contact with the cutting region 34, and hence unintended cutting by ultrasonic vibration can be inhibited. Thus, also in a narrow space S, the cutting region 34 that is a blade region appropriately comes in contact with the treatment target Ap, and a treatment performance in the treatment of cutting the treatment target Ap is acquired.

Also in this embodiment, similarly to the first and second embodiments, the gap (space) G formed by the second extending surface 62 of the bend extending portion 32 and the second relay surface 52 of the narrowing portion 31 is formed on the proximal side of the cutting region 34. Consequently, when the cutting region 34 of the treatment portion 33 is brought into contact with the affected part Ap from an opening So of the narrow space S of a joint cavity on the left side of FIG. 9A, a position in the vicinity of the opening So of the narrow space S of the joint cavity is hard to interfere with the second extending surface 62. Furthermore, as shown in FIG. 10A, also when the cutting region 34 of the treatment portion 33 is brought into contact with the affected part Ap at a position inner than the position shown in FIG. 9A from the opening So of the narrow space S of the joint cavity on the left side of FIG. 10A, the position in the vicinity of the opening So of the narrow space S of the joint cavity is hard to interfere with the narrowing portion 31.

Consequently, in the ultrasonic probe 8 according to the present embodiment, the bend extending portion 32 is provided on the distal side of the narrowing portion 31. The bend extending portion 32 extends in a state of bending to the second intersecting direction P2 side of the longitudinal axis C. Furthermore, in the bend extending portion 32, the cutting region 34 is formed toward the second intersecting direction P2 side. Due to the above-mentioned constitution, a region of the ultrasonic probe 8 excluding the cutting region 34 is prevented from interfering with the tissue or the like other than the treatment target Ap (e.g., an area other than the affected part Ap in a bone Ba) also in the narrow space S of the joint cavity in which an angle range of an approach angle to the treatment target Ap is limited to a small range. Consequently, the position of the treatment target Ap excluding an area in contact with the cutting region 34 is hard to come in contact with the ultrasonic probe 8, and hence unintended cutting by the ultrasonic vibration can be inhibited. In consequence, also in the narrow space S, the cutting region 34 that is the blade region appropriately comes in contact with the treatment target Ap, and the treatment performance in the treatment of cutting the treatment target Ap is acquired.

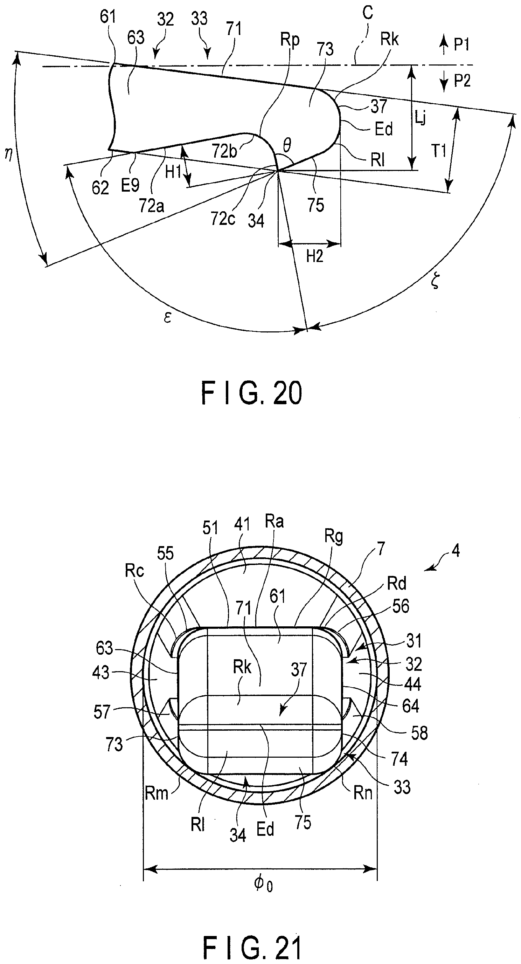

Furthermore, in the present embodiment, as shown in FIG. 21, the narrowing portion 31, the bend extending portion 32 and the treatment portion 33 are arranged in a range inner than a minimum inner diameter φo of a sheath 7 in projection from the distal side. Consequently, in the narrow space S of the joint cavity, the region other than the cutting region 34 in the ultrasonic probe 8 is further effectively prevented from interfering with the tissue or the like other than the treatment target Ap. Consequently, in the narrow space S, the cutting region 34 that is the blade region further appropriately comes in contact with the treatment target Ap.

The present embodiment has a constitution where the narrowing portion 31, the bend extending portion 32 and the treatment portion 33 are arranged in the range inner than the minimum inner diameter φo of the sheath 7. Consequently, it is easy to insert the ultrasonic probe 8 through the sheath 7. In consequence, time and labor in assembling an ultrasonic treatment instrument 2 are decreased.

FIG. 21 is a view of the sheath 7 and the ultrasonic probe 8 which are seen from the distal side. The minimum inner diameter φo of the sheath 7 shown in FIG. 21 is larger than an outer diameter φa of the ultrasonic probe 8 at the boundary position E1 between the probe main body 15 and the narrowing portion 31. In an example where the outer diameter φa is 4. 9 mm, the minimum inner diameter φo of the sheath 7 is, for example, 5 mm. In the projection seen from the distal side, the treatment portion 33, the bend extending portion 32 and the narrowing portion 31 are arranged in the range inner than the minimum inner diameter φo of the sheath 7.

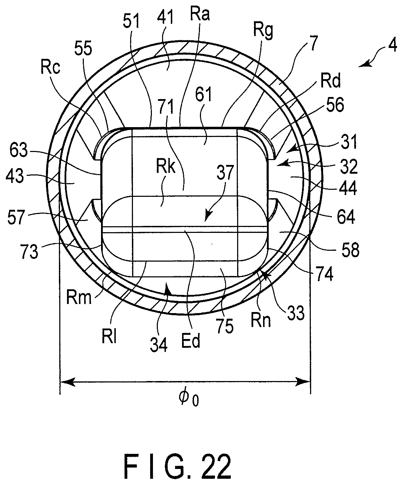

The treatment portion 33 of the ultrasonic probe 8 is not limited to the example shown in FIG. 21, and it is also preferable that the treatment portion is formed as in a modification shown in FIG. 22.

FIG. 22 shows a state where in the projection seen from the distal side of the longitudinal axis C, the narrowing portion 31, the bend extending portion 32 and the treatment portion 33 are arranged in a range inner than the outer diameter φa of the ultrasonic probe 8 at the boundary position E1. Furthermore, the narrowing portion 31, the bend extending portion 32 and the treatment portion 33 are capable of performing the treatment as described in the first embodiment, even in the state where the portions are arranged in the range inner than the outer diameter φa of the ultrasonic probe 8 at the boundary position E1 as shown in FIG. 22. Therefore, as long as the portions are arranged in a range inner than the inner diameter φo of the sheath 7, the treatment portion 33 may have a size to be in contact with the outer diameter φa of the ultrasonic probe 8.

### (Fourth Embodiment)

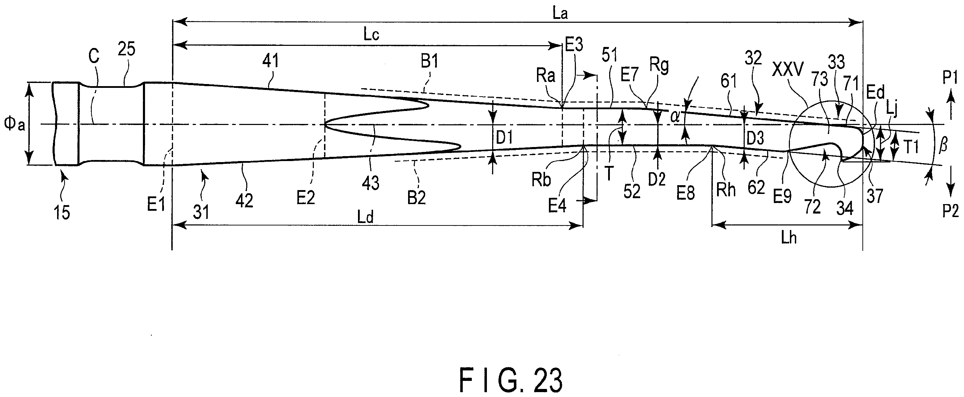

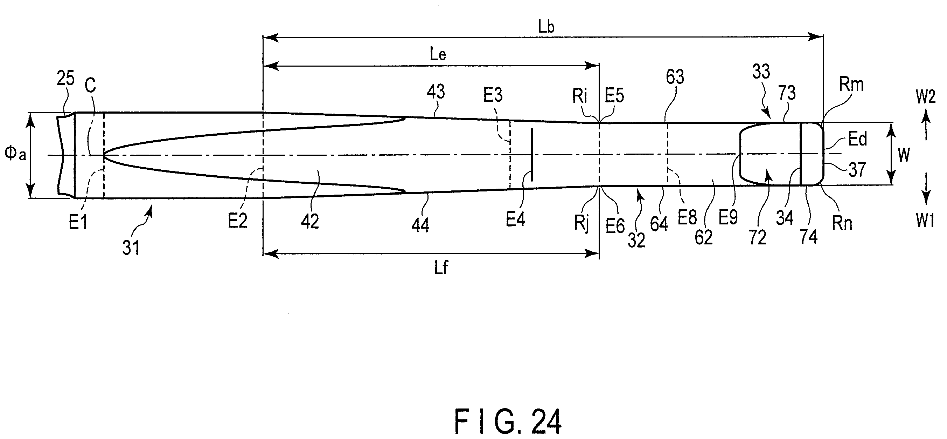

Next, a fourth embodiment will be described with reference to FIG. 23 to FIG. 26. This embodiment is a modification of the first to third embodiments, the same members or members having the same functions as in the members described in the first to third embodiments are denoted with the same signs to the utmost, and detailed description is omitted. This embodiment is especially a modification of the third embodiment.

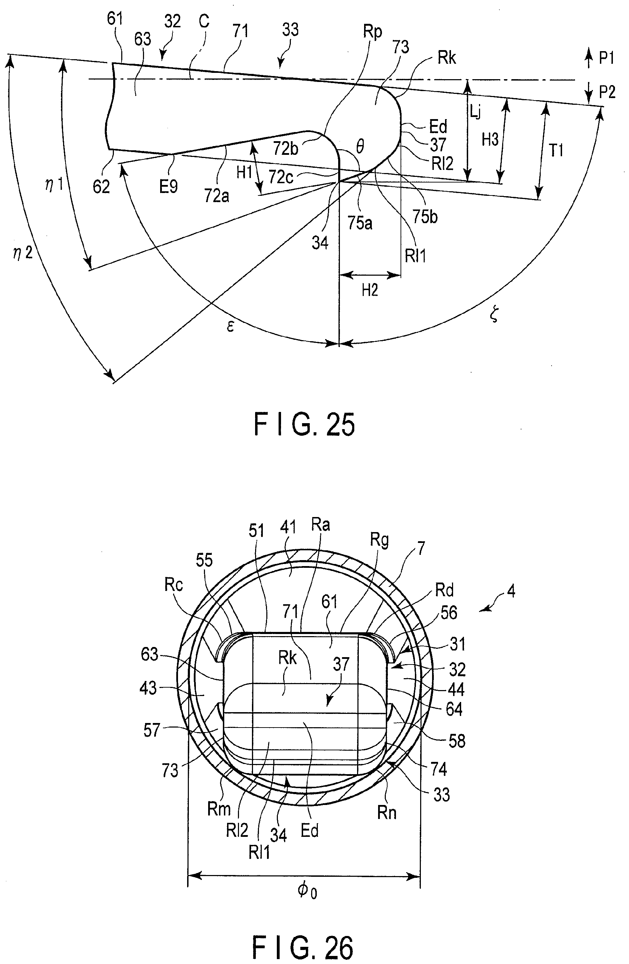

As shown in FIG. 25, the flat portion 75 described in the third embodiment has a first flat portion 75a adjacent to a cutting region 34, and a second flat portion 75b adjacent to the first flat portion 75a on a side close to a distal end Ed of a treatment portion 33 due to a curved surface 72b having a radius Rp.

As shown in FIG. 23 and FIG. 25, in this embodiment, at least a part of the cutting region 34 is present at a position far from a longitudinal axis C to a virtually extending plane of a second extending surface 62. Consequently, in the cutting region 34, when the first extending surface 62 virtually extends, the plane intersects a second flat portion 72c adjacent to a proximal side of the cutting region 34 and intersects at least one of the first and second flat portions 75a and 75b which are adjacent to a distal side of the cutting region 34. In other words, the cutting region 34 projects from the virtually extending plane of the first extending surface 62 to a second intersecting direction P2 side. Thus, differently from the example of the third embodiment, a projecting length of the cutting region 34 from the second extending surface 62 may be adjusted.

It is to be noted that a distance between a first relay surface 51 and a second relay surface 52, i.e., a thickness T of a distal portion of a narrowing portion 31 is 1.7 mm in a certain example. In this case, a distance between the longitudinal axis C and the first relay surface 51 is 0.75 mm, and a distance D2 between the longitudinal axis C and the second relay surface 52 is 0.95 mm. Consequently, a center of gravity of the distal portion of the narrowing portion 31 shifts to the second intersecting direction P2 side of the longitudinal axis C in the same manner as in the third embodiment.

It is to be noted that each of radiuses Rc, Rd, Re and Rf is 0.5 mm, each of radiuses Rk, Rl1, Rl2, Rm and Rn is 0.5 mm, and a radius Rp is 0.5 mm.

Furthermore, as one example, a dimension La is 32 mm, a dimension Lb is 25 mm, a dimension Lc is 18 mm, a dimension Ld is 19 mm, and each of dimensions Le and Lf is 15 mm. A distance Lh is 7 mm. A height H1 is 0.9 mm, a distance Lk (= a height H2) is 1 mm, and a height H3 is 1.4 mm. Furthermore, a thickness T1 between a distal end of a first continuous surface 71 of the treatment portion 33 and the cutting region 34 is 1.6 mm, and a width W is 2.8 mm.

An angle η1 is determined by a first extending surface 61 and the first continuous surface 71 which are directed to a first intersecting direction P1 side and the first flat portion 75a adjacent to the distal side of the cutting region 34. An angle η2 is determined by the first extending surface 61, the first continuous surface 71, and the second flat portion 75b adjacent to the distal side of the first flat portion 75a. In the certain example, the angle η1 is 25° and the angle η2 is 45°. It is to be noted that in the certain example, each of angles α and P is 5°, an angle ε is 80°, and an angle ζ is 85°. Thus, differently from the third embodiment, an angle of a bend extending portion 32 may be adjusted. The above-mentioned protruding length and angles α and β are adjusted, so that the treatment portion 33 suitable for a joint of a treatment target can be formed.

Here is described a shape of a range (a range shown with a broken line B2 in FIG. 23) of a second narrowing outer surface 42, the second relay surface 52 and the second extending surface 62 on the second intersecting direction P2 side in the ultrasonic probe 8. In the second narrowing outer surface 42 of the narrowing portion 31, a distance (a first distance) D1 on the second intersecting direction P2 side which is perpendicular to the straight longitudinal axis C increases from the distal side toward the proximal side along the longitudinal axis C. In a region between a distal end of the second relay surface 52 of the narrowing portion 31 (a boundary position E8) and a proximal end thereof (a boundary position E4), the distance (a second distance) D2 to the longitudinal axis C is smaller than the distance D1 and uniform. The distance D2 to the longitudinal axis C is also uniform at a distal end of the second narrowing outer surface 42 (the boundary position E4) and a proximal end of the second extending surface 62 (the boundary position E8). In the second extending surface 62 of the bend extending portion 32, a distance D3 (> D2) to the longitudinal axis C on the second intersecting direction P2 side increases toward the distal side. Furthermore, a distance Lj between the longitudinal axis C and the cutting region 34 is larger than the distance D3 at any position, though an example of a specific numeric value is omitted. Consequently, as described above, the cutting region 34 is provided at a position more away from the longitudinal axis C than the bend extending portion 32 in the second intersecting direction P2. Therefore, according to the ultrasonic probe 8 of this embodiment, in the state where the cutting region 34 is in contact with a treatment target Ap, it is easy to form a gap (space) G (see FIG. 9A and FIG. 10A) between a tissue or the like adjacent to the proximal side of the cutting region 34 and each of the second relay surface 52 and the second extending surface 62. The gap G can contribute to inhibition of interference with the tissue or the like adjacent to the proximal side of the cutting region 34.

FIG. 26 is a view of a sheath 7 and the ultrasonic probe 8 which are seen from the distal side. A minimum inner diameter φo of the sheath 7 shown in FIG. 26 is larger than an outer diameter φa of the ultrasonic probe 8 at a boundary position E1 between a probe main body 15 and the narrowing portion 31. In an example where the outer diameter φa is 3.8 mm, the minimum inner diameter φo of the sheath 7 is, for example, 4 mm. In projection seen from the distal side, the treatment portion 33, the bend extending portion 32 and the narrowing portion 31 are arranged in a range inner than the minimum inner diameter φo of the sheath 7.

Although not shown in the drawing, it is also preferable that in the projection seen from the distal side, the treatment portion 33, the bend extending portion 32 and the narrowing portion 31 are arranged in a range inner than the outer diameter φa of the ultrasonic probe 8 at the boundary position E1.

Also when the ultrasonic probe 8 is formed in this way, the ultrasonic probe is usable in the same manner as described in the third embodiment.

### (Fifth Embodiment)

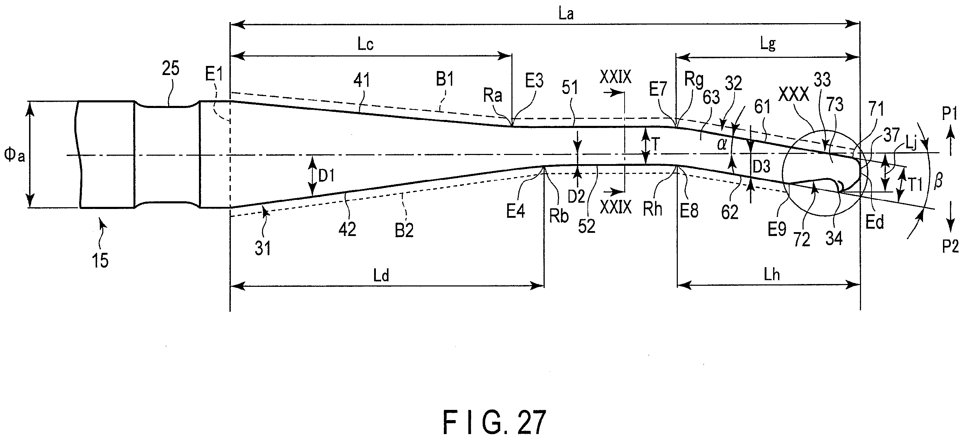

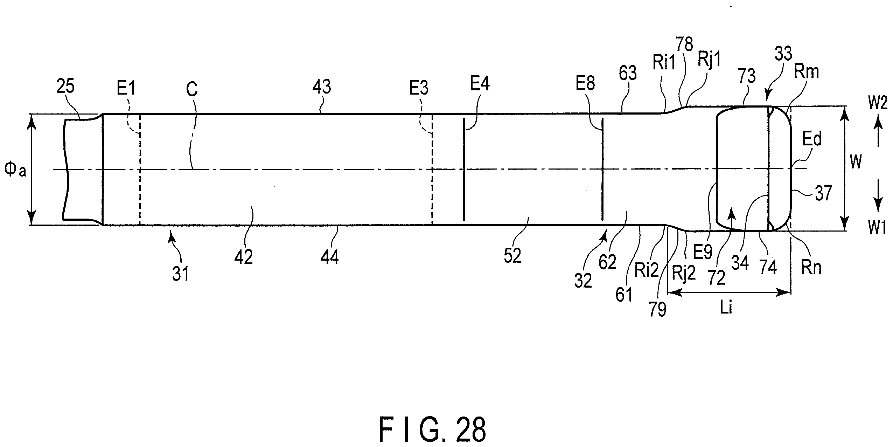

Next, a fifth embodiment will be described with reference to FIG. 27 to FIG. 30. This embodiment is a modification of the first to fourth embodiments, the same members or members having the same functions as in the members described in the first to fourth embodiments are denoted with the same signs to the utmost, and detailed description is omitted. This embodiment is especially a modification of the third embodiment.

Here, a position in the vicinity of a distal portion of a narrowing portion 31 shifts. In other words, a dimension Ld is longer than a dimension Lc. In a certain example, the dimension Lc is 13 mm and the dimension Ld is 14.5 mm. Consequently, boundary positions E3 and E4 shift forward and backward along a longitudinal axis C. In particular, the boundary position E3 of a first narrowing outer surface 41 is disposed closer to a proximal side than the boundary position E4 of a second narrowing outer surface 42. Furthermore, a dimension between a first relay surface 51 and the longitudinal axis C is larger than a dimension D2 between a second relay surface 52 and the longitudinal axis C. A distance between the first relay surface 51 and the second relay surface 52, i.e., a thickness T of the distal portion of the narrowing portion 31 is 1.75 mm in the certain example. In this case, a distance between the longitudinal axis C and the first relay surface 51 is 1.25 mm and the distance D2 between the longitudinal axis C and the second relay surface 52 is 0.5 mm. Consequently, a center of gravity of the distal portion of the narrowing portion 31 shifts to a first intersecting direction P1 side of the longitudinal axis C.

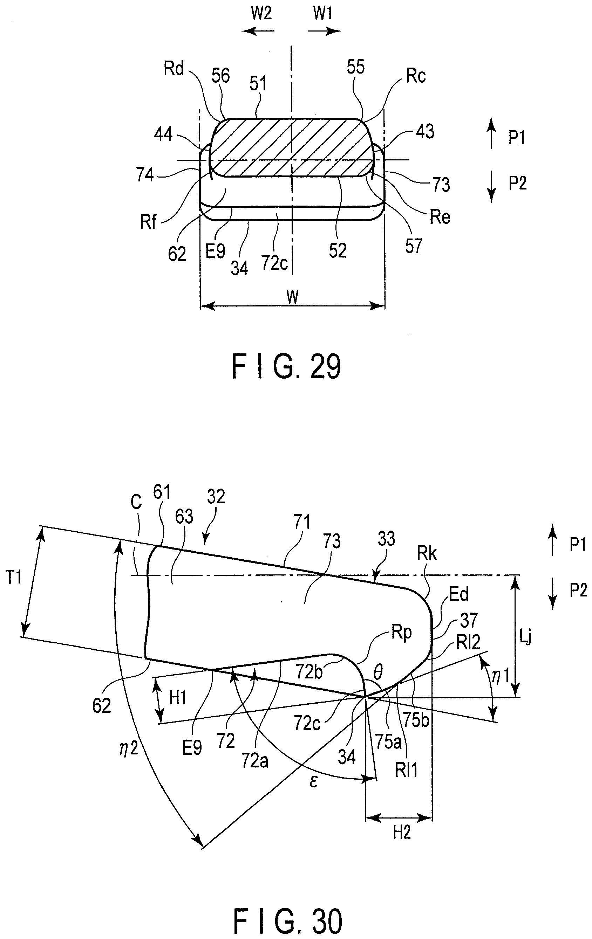

As shown in FIG. 29, a treatment portion 33 shifts to a second intersecting direction P2 side. Thus, the distal portion of the narrowing portion 31 shifts to the first intersecting direction P1 side, whereby balance between the first intersecting direction P1 and the second intersecting direction P2 of a bend extending portion 32 and the treatment portion 33 is taken. Therefore, in an ultrasonic probe 8 according to the present embodiment, there is inhibited transverse vibration (improper vibration) in a vibrating direction substantially parallel to the first intersecting direction P1 and the second intersecting direction P2.

Here is described a shape of a range (a range shown with a broken line B2 in FIG. 27) of the second narrowing outer surface 42, the second relay surface 52 and a second extending surface 62 on the second intersecting direction P2 side in the ultrasonic probe 8. In the second narrowing outer surface 42 of the narrowing portion 31, a distance (a first distance) D1 on the second intersecting direction P2 side which is perpendicular to the straight longitudinal axis C increases from the distal side toward the proximal side along the longitudinal axis C. In a region between a distal end of the second relay surface 52 of the narrowing portion 31 (a boundary position E8) and a proximal end thereof (the boundary position E4), the distance (a second distance) D2 to the longitudinal axis C is smaller than a distance D1 and uniform. The distance D2 to the longitudinal axis C is also uniform at a distal end of the second narrowing outer surface 42 (the boundary position E4) and a proximal end of the second extending surface 62 (the boundary position E8). In the second extending surface 62 of the bend extending portion 32, a distance D3 (> D2) to the longitudinal axis C on the second intersecting direction P2 side increases toward the distal side. Furthermore, a distance Lj between the longitudinal axis C and a cutting region 34 is larger than the distance D3 at any position, though an example of a specific numeric value is omitted. Consequently, as described above, the cutting region 34 is provided at a position more away from the longitudinal axis C than the bend extending portion 32 in the second intersecting direction P2. Therefore, according to the ultrasonic probe 8 of this embodiment, in the state where the cutting region 34 is in contact with a treatment target Ap, it is easy to form a gap (space) G (see FIG. 9A and FIG. 10A) between a tissue or the like adjacent to the proximal side of the cutting region 34 and each of the second relay surface 52 and the second extending surface 62. The gap G can contribute to inhibition of interference with the tissue or the like adjacent to the proximal side of the cutting region 34.

It is to be noted that as one example, each of radiuses Rc, Rd, Re and Rf is 0.5 mm. Here, in the same manner as described in the second embodiment, suitable radiuses Ri1, Ri2, Rj1 and Rj2 are prescribed. Furthermore, a radius Rk is 0.5 mm, a radius Rl1 is 1 mm, a radius R12 is 0.4 mm, each of radiuses Rm and Rn is 1 mm, and a radius Rp is 0.5 mm.

The dimension Lb prescribed in the third and fourth embodiments is not prescribed here. This is because in the present embodiment, a distance from the longitudinal axis C to an outer surface directed in a width direction W is uniform from a distal end of a supported portion 25 to the bend extending portion 32. Furthermore, dimensions Le and Lf are not prescribed similarly. As one example, a dimension La is 29.2 mm, the dimension Lc is 13 mm, and the dimension Ld is 14.5 mm. Each of distances Lg and Lh is 8.5 mm. A distance Li is 5.5 mm. A height H1 is 0.7 7 mm, and a distance Lk (= a height H2) is 1 mm. A thickness T1 between a distal end of a first continuous surface 71 of the treatment portion 33 and the cutting region 34 is 1.7 mm, and a width W of the treatment portion 33 is 5.5 mm.

In the certain example, each of angles α and β is 10°, an angle ε is 90°, an angle η1 is 30°, and an angle η2 is 50°.

Hitherto, the embodiments and the like of the present invention have been described, but the present invention is not limited to the above-mentioned embodiments and the like, and needless to say, various modifications can be achieved without departing from the gist of the invention.

## Claims

1. An ultrasonic probe for arthroscopic surgery which is for use in an operation of a joint and which is configured to transmit ultrasonic vibration from a proximal side to a distal side thereof, the ultrasonic probe comprising:
a probe main body which extends from the proximal side to the distal side along a linear longitudinal axis and to which an ultrasonic transducer to generate the ultrasonic vibration is connected to the proximal side;
a narrowing portion which is continuous with the distal side of the probe main body and whose sectional area vertical to the longitudinal axis decreases from the proximal side toward the distal side;
a bend extending portion which is provided on the distal side of the narrowing portion and which extends in a state of bending in an intersecting direction to the longitudinal axis when the intersecting direction intersecting the longitudinal axis is prescribed; and
a treatment portion which is provided on the distal side of the bend extending portion, and which includes a cutting region that is configured to cut a bone or a cartilage by use of the ultrasonic vibration in the joint at a position more away from the longitudinal axis than the bend extending portion in the intersecting direction,
wherein in projection seen from the distal side, the narrowing portion, the bend extending portion and the treatment portion are arranged in a range inner than a minimum inner diameter of a sheath into which the ultrasonic probe is inserted.

2. The ultrasonic probe of claim 1, wherein:
the bend extending portion includes a first extending surface approaching or intersecting the longitudinal axis from the proximal side toward the distal side, and a second extending surface disposed away from the longitudinal axis, from the proximal side toward the distal side, and
the cutting region of the treatment portion is provided on a side of the second extending surface.

3. The ultrasonic probe of claim 2, wherein at least a part of the cutting region of the treatment portion is present on a virtually extending plane of the second extending surface.

4. The ultrasonic probe of claim 3,
wherein the cutting region of the treatment portion is in the form of a hook.

5. The ultrasonic probe of claim 2, wherein at least a part of the cutting region of the treatment portion is present at a position far from the longitudinal axis on a virtually extending plane of the second extending surface.

6. The ultrasonic probe of claim 1, wherein the cutting region of the treatment portion is in the form of a file.

7. The ultrasonic probe of claim 6, wherein the file-shaped cutting region is formed as a part of a spherical surface.

8. The ultrasonic probe of claim 1, wherein:
the narrowing portion includes a narrowing outer surface on a side of the intersecting direction, and
the bend extending portion includes an extending surface disposed away from the longitudinal axis, from the proximal side toward the distal side along the longitudinal axis on the side of the intersecting direction,
the ultrasonic probe comprising a relay surface continuous with the distal side of the narrowing outer surface and continuous with the proximal side of the extending surface,
wherein:
in the narrowing outer surface, a first distance to the longitudinal axis on the side of the intersecting direction increases from the distal side toward the proximal side along the longitudinal axis,
in a region between a distal end of the relay surface and a proximal end thereof, a second distance to the longitudinal axis on the side of the intersecting direction is equal to or smaller than the first distance of the narrowing outer surface, and
in the extending surface of the bend extending portion, a third distance to longitudinal axis on the side of the intersecting direction increases from the proximal side toward the distal side, and the third distance to the longitudinal axis is equal to or larger than the second distance.

9. The ultrasonic probe of claim 8, wherein the second distance is uniform between the distal end of the relay surface and the proximal end thereof.

10. The ultrasonic probe of claim 1, wherein in the projection seen from the distal side, the narrowing portion, the bend extending portion and the treatment portion are arranged in a range inner than an outer diameter of the ultrasonic probe at a boundary position between the probe main body and the narrowing portion.

11. An ultrasonic probe unit for arthroscopic surgery comprising:
the ultrasonic probe for the arthroscopic surgery according to claim 1; and
a sheath into which the probe main body of the ultrasonic probe is inserted,
wherein in the projection seen from the distal side, the narrowing portion, the bend extending portion and the treatment portion are arranged in a range inner than a minimum inner diameter of the sheath.
